# EUROPEAN PATENT SPECIFICATION

(11) **EP 4 408 512 B1**
(45) Date of publication and mention of the grant of the patent: **30.07.2025**
(21) Application number: 22802514.4
(22) Date of filing: 27.09.2022
(51) Int. Cl.: A61M 16/06, A61M 16/08

(54) **TUBING ASSEMBLY FOR PATIENT INTERFACE DEVICE**
SCHLAUCHSYSTEM FÜR PATIENTENSCHNITTSTELLENVORRICHTUNG
ENSEMBLE DE TUBES POUR DISPOSITIF D'INTERFACE DU PATIENT

(30) Priority: 28.09.2021 US 202163249106 P
(43) Date of publication of application: 07.08.2024
(73) Proprietor: Koninklijke Philips N.V., 5656 AG Eindhoven (NL)
(72) Inventor: HIMES, Kevin Daniel, 5656 AG Eindhoven (NL); IGNACZAK, Dave, 5656 AG Eindhoven (NL); MCCASLIN, Jonathan, 5656 AG Eindhoven (NL)
(74) Representative: Philips Intellectual Property & Standards
(86) International application number: PCT/EP2022/076797
(87) International publication number: WO 2023/052342

(56) References cited:
- WO-A1-2005/099801
- WO-A1-2011/022779
- WO-A1-2017/124152
- WO-A1-2019/185474
- WO-A1-2022/016220
- US-A1- 2017 312 469

## Description

### BACKGROUND OF THE INVENTION

### 1. Field of the Invention

The present invention relates generally to a tubing assembly for use with patient interface devices and enhancements thereto.

### 2. Description of the Related Art

During medical treatments, it may be sometimes desirable to deliver a flow of breathing gas non-invasively to an airway of a patient, i.e., without intubating the patient or surgically inserting a tracheal tube in their esophagus. For example, it is known to ventilate a patient using a technique known as non-invasive ventilation. It is also known to deliver continuous positive airway pressure (CPAP) or variable airway pressure, which varies with patient's respiratory cycle, to treat a medical disorder, such as sleep apnea syndrome, in particular, obstructive sleep apnea (OSA), or congestive heart failure.

Non-invasive ventilation and pressure support therapies may involve the placement of a patient interface device in combination with a tubing assembly on a head of the patient. The patient interface device may comprise, without limitation, a nasal mask that covers the patient's nose, a nasal cushion having nasal prongs that are received within the patient's nares, a nasal/oral mask that covers the nose and mouth, or a full face mask that covers the patient's face. The patient interface device interfaces the ventilator or pressure support device with the airway of the patient, so that a flow of breathing gas can be delivered from a pressure/flow generating device to the airway of the patient.

A number of known tubing assemblies provide airflow to the patient via one or more delivery tubes that wrap around portions of the head of the patient as part of the tubing assembly. The tubing assembly generally comprises a manifold. The manifold is coupled to, and in fluid communication with, a delivery conduit. The delivery conduit is further coupled to, and in fluid communication with, the pressure/flow generating device.

Such known patient interface devices, however, have a number of drawbacks. For example, due to the functionality of the tubing assembly in providing the airflow to the patient, sizing options of such tubing assembly may be typically limited and generally not customizable. In order to fit different head sizes, options of different frame sizes exist. In some cases, such multiple frame sizes may be accomplished through individual parts. The individual parts may pose multiple challenges in ensuring that the patient receives an appropriate frame size. In some cases, the individual parts may have to be assembled/disassembled by the patient or a caregiver prior to use to adjust the tubing assembly according to a patient's requirement. Such an approach may be confusing for the patient and may affect ease of use. Further, the individual parts of the tubing assemblies may increase inventory, part numbers, and a cost of the tubing assembly. In some cases, some of the individual parts may get misplaced. In some other cases, as the multiple frame sizes are accomplished through the individual parts, the tubing assembly may be bulky to handle and wear. Thus, such tubing assemblies may not be easy to use and may not be easily adjusted by the patients or care givers according to patient requirements.
WO 2017/124152 A1 discloses an inflatable positioning and stabilizing structure for a patient interface for delivery of a flow of pressurized air to an entrance of a patient's airways.
WO 2019/185474 A1 discloses a frame for use in an interface device for delivering a flow of treatment gas to the airway of a patient.
WO 2011/022779 A1 discloses systems, devices, and methods for treatment of respiratory disease or sleep disordered breathing.
WO 2022/016220 A1, which is novelty-only prior art pursuant to Article 54(3) EPC, discloses a positioning and stabilizing structure comprising a gas delivery tube to receive a flow of air from a connection port on top of the patients head and to deliver the flow of air to the entrance of the patients airways.

### SUMMARY OF THE INVENTION

The invention is defined in the appended independent claim 1. Preferred embodiments are matter of the dependent claims.

Accordingly, a first aspect of the present invention relates to a tubing assembly for use with a patient interface device in delivering a flow of breathing gas to an airway of a user. The tubing assembly comprises a manifold portion structured to be disposed generally atop the user's head and adapted to be coupled to a conduit carrying the flow of breathing gas. The tubing assembly also comprises a plurality of tubular portions. Each tubular portion extends from the manifold portion to a distal end which is structured to be coupled to the patient interface device. Each tubular portion is structured to communicate the flow of breathing gas from the manifold portion to the patient interface device. The tubing assembly further comprises at least one adjustment unit configured to adjust a length of a corresponding tubular portion from the plurality of tubular portions. The at least one adjustment unit comprises a first adjustment member integral with or fixedly connected to the corresponding tubular portion and a second adjustment member integral with or fixedly connected to the corresponding tubular portion. The first adjustment member and the second adjustment member are movable relative to each other. The first adjustment member and the second adjustment member are selectively and releasably locked to each other at a selected discrete position from a plurality of discrete positions. The plurality of discrete positions corresponds to a plurality of predetermined lengths of the corresponding tubular portion.

A second aspect of the present invention relates to a respiratory interface system for use in delivering a flow of breathing gas to an airway of a user. The respiratory interface system comprises a patient interface device structured to sealingly engage the airway of the user. The respiratory interface system further comprises the tubing assembly of the first aspect. The distal end of each tubular portion is coupled to the patient interface device.

A third aspect of the present disclosure relates to a method for delivering a flow of breathing gas to an airway of a user (the method not being separately claimed). The method comprises receiving, via a manifold portion, a flow of breathing gas from a conduit. The method also comprises communicating, via a tubular portion, the flow of breathing gas from the manifold portion to a patient interface device that is sealingly engaged with the airway of the user. The method further comprises adjusting, via at least one adjustment unit, a length of the tubular portion. The method further comprises selectively and releasably locking the at least one adjustment unit at a selected discrete position from a plurality of discrete positions. The plurality of discrete positions corresponds to a plurality of predetermined lengths of the tubular portion.

A fourth aspect of the present disclosure relates to a tubing assembly for use with a patient interface device in delivering a flow of breathing gas to an airway of a user. The tubing assembly comprises manifold means for receiving a flow of breathing gas from a conduit. The tubing assembly also comprises tubular means for communicating the flow of breathing gas from the manifold means to the patient interface device that is sealingly engaged with the airway of the user. The tubing assembly further comprises adjustment means for adjusting a length of the tubular means. The tubing assembly further comprises locking means for selectively and releasably locking the adjustment means at a selected discrete position from a plurality of discrete positions. The plurality of discrete positions corresponds to a plurality of predetermined lengths of the tubular means.

A general object of the present invention is to provide a tubing assembly for use with a patient interface device in delivering a flow of breathing gas to an airway of a user that may be adjusted to various discrete positions.

Another object of the present invention is to provide a tubing assembly that can be used by users of different head sizes.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the scope of the appended claims.

### BRIEF DESCRIPTION OF THE DRAWINGS

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.
FIG. 1A is a perspective view of a respiratory interface system disposed on a user, according to an exemplary embodiment of the present invention;
FIGS. 1B to 1D are partially schematic views of respiratory interface systems, according to exemplary embodiments of the present invention;
FIG. 2 is a perspective view of a tubing assembly associated with the respiratory interface system of FIG. 1A, according to a first embodiment of the present invention;
FIG. 3A is an exploded view of a pair of adjustment units of the tubing assembly shown in FIG. 2, according to the first embodiment of the present invention;
FIG. 3B is a bottom perspective view illustrating a first adjustment member and a second adjustment member of the tubing assembly shown in FIG. 2, according to the first embodiment of the present invention;
FIG. 4A is a perspective view illustrating an adjustment section in an extended position, according to the first embodiment of the present invention;
FIG. 4B is a perspective view illustrating the adjustment section in an intermediate position, according to the first embodiment of the present invention;
FIG. 4C is a perspective view illustrating the adjustment section in a contracted position, according to the first embodiment of the present invention;
FIG. 5 is a perspective view of a tubing assembly associated with the respiratory interface system of FIG. 1A, according to a second embodiment of the present invention;
FIG. 6A is an exploded view of a portion of the tubing assembly shown in FIG. 5, according to the second embodiment of the present invention;
FIG. 6B is a cross-sectional view of a second adjustment member of the tubing assembly of FIG. 6A along a plane A-A1, according to the second embodiment of the present invention;
FIG. 7A is a perspective view of the tubing assembly of FIG. 5 in an extended position, according to the second embodiment of the present invention;
FIG. 7B is a perspective view of the tubing assembly of FIG. 5 in an intermediate position, according to the second embodiment of the present invention;
FIG. 7C is a perspective view of the tubing assembly of FIG. 5 in a contracted position, according to the second embodiment of the present invention; and,
FIG. 8 is a flowchart for a method for delivering a flow of breathing gas to an airway of the user.

### DETAILED DESCRIPTION OF EXEMPLARY EMBODIMENTS

In the following description, reference is made to the accompanying figures that form a part thereof and in which various embodiments are shown by way of illustration. It is to be understood that other embodiments are contemplated and may be made without departing from the scope or spirit of the appended claims. The following detailed description, therefore, is not to be taken in a limiting sense.

As used herein, the singular form of "a", "an", and "the" include plural references unless the context clearly dictates otherwise.

As used herein, the statement that two or more parts or components are "coupled" shall mean that the parts are joined or operate together either directly or indirectly, i.e., through one or more intermediate parts or components, so long as a link occurs.

As used herein, "fixedly connected" or "fixed" means that two components are coupled so as to move as one while maintaining a constant orientation relative to each other.

As used herein, the phrase "sealingly engage" shall mean elements which contact each other in a manner such that a generally air-tight seal is formed therebetween.

As used herein, the term "integral" means a component is created as a single piece or unit. That is, a component that includes pieces that are created separately and then coupled together as a unit is not a "unitary" component or body.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

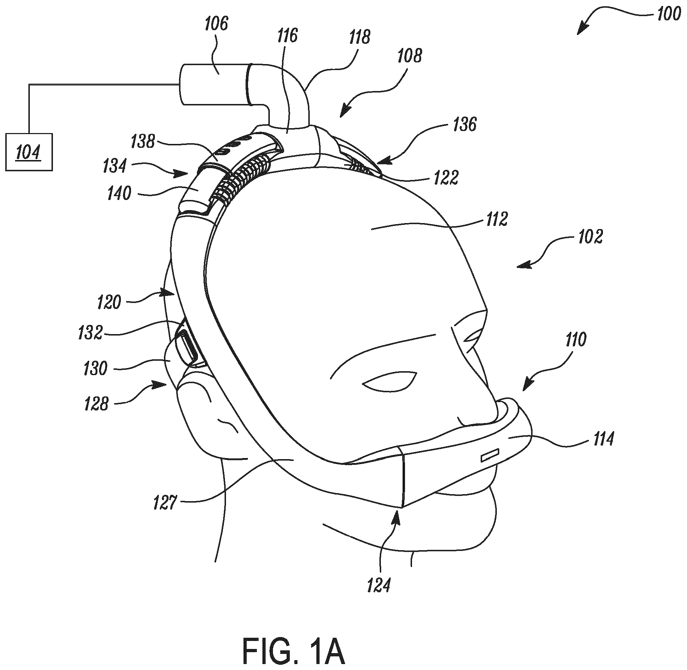

FIG. 1A illustrates a respiratory interface system 100 for use in delivering a flow of breathing gas to an airway of a user 102, according to an exemplary embodiment of the present invention. Respiratory interface system 100 may be adapted to provide a regimen of respiratory therapy to user 102. Respiratory interface system 100 comprises a pressure generating device 104 (shown schematically), a conduit 106 fluidly coupled to a tubing assembly 108, and a patient interface device 110 fluidly coupled to tubing assembly 108. Pressure generating device 104 is structured to generate a flow of positive pressure breathing gas and may comprise, without limitation, ventilators, constant pressure support devices (such as a continuous positive airway pressure device, or CPAP device), variable pressure devices (e.g., BiPAP^{®}, Bi-Flex^{®}, or C-Flex^{™} devices manufactured and distributed by Philips Respironics of Murrysville, Pa.), and auto-titration pressure support devices. Conduit 106 is structured to communicate the flow of breathing gas from pressure generating device 104 to patient interface device 110 through tubing assembly 108. Specifically, the breathing gas enters at a top of a head 112 of user 102. Conduit 106, tubing assembly 108, and patient interface device 110 are often collectively referred to as a user circuit.

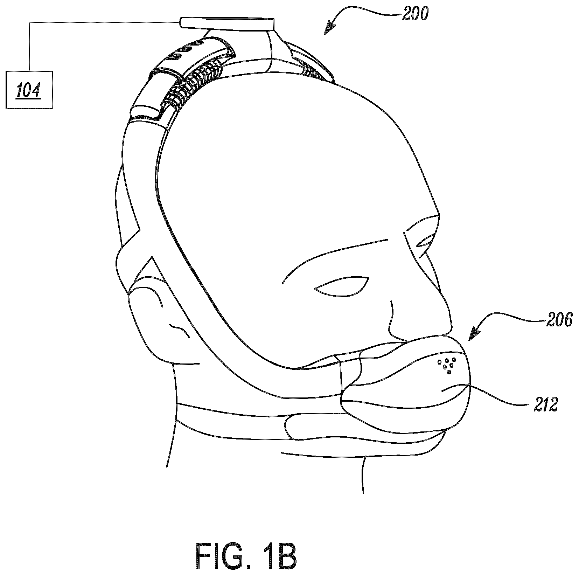

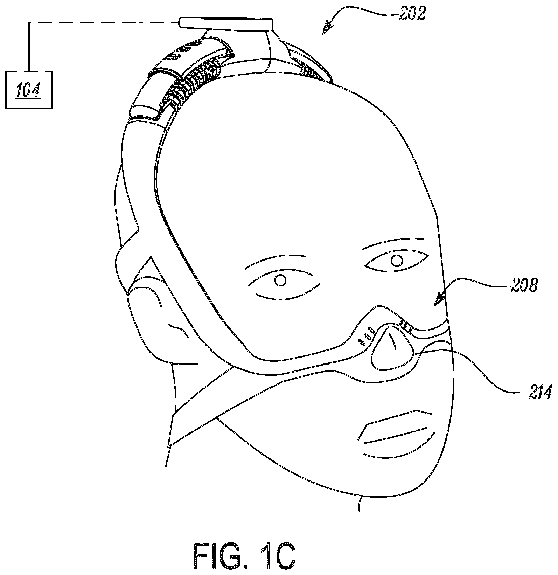

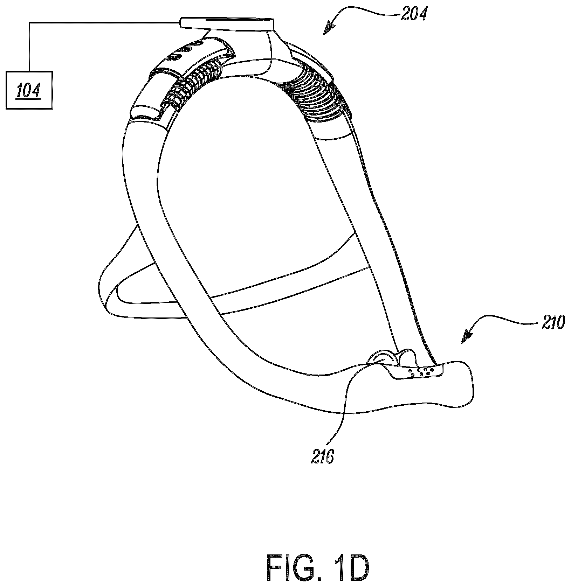

Further, patient interface device 110 is structured to sealingly engage the airway of user 102. Patient interface device 110 comprises a patient sealing element 114. In an exemplary embodiment, patient sealing element 114 may comprise a nasal cushion made of a soft, flexible material, such as, without limitation, silicone, an appropriately soft thermoplastic elastomer, a closed-cell foam, or any other suitable material or combination of such materials. It is to be appreciated, however, that any type of patient sealing element 114, such as a nasal/oral mask, a nasal pillow, or a full face mask, which may facilitate a delivery of the flow of breathing gas to the airway of user 102, may be used as patient sealing element 114 while remaining within the scope of the appended claims. It should be noted that tubing assembly 108, in conjunction with additional attachments, may allow coupling of different types of patient interface devices, without any limitations. FIGS. 1B, 1C, and 1D show exemplary embodiments of other tubing assemblies 200, 202, 204, respectively, of similar construction as tubing assembly 108 except used in conjunction with respective patient interface devices 206, 208, 210 having respective patient sealing elements 212, 214, 216 in the form of: a full face (under the nose) cushion (FIG. 1B), a nasal (over the nose) cushion (FIG. 1C), and individual nasal pillows (FIG. 1D).

Further, as shown in FIG. 1A, respiratory interface system 100 comprises tubing assembly 108 for use with patient interface device 110 in delivering the flow of breathing gas to the airway of user 102. Tubing assembly 108 may be interchangeably referred to as tubing means 108.

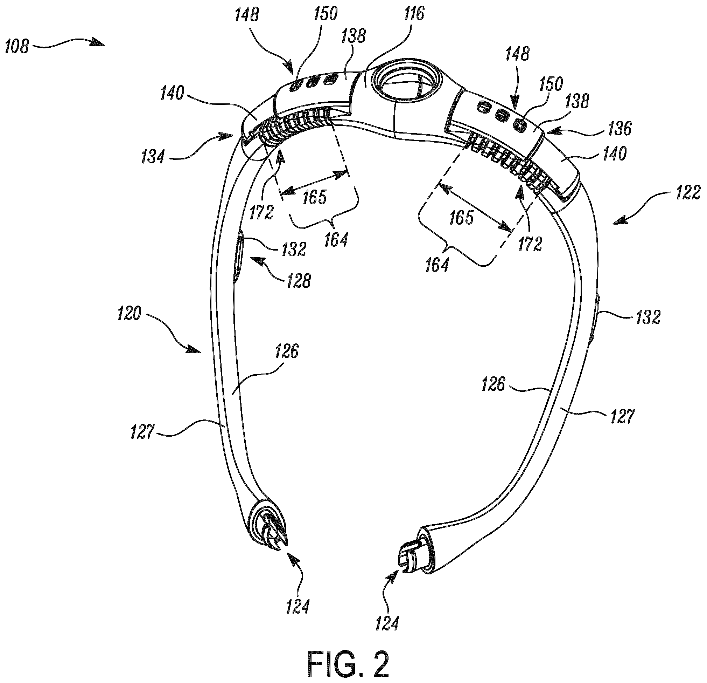

With reference to FIGS. 1A and 2, tubing assembly 108 comprises a manifold portion 116 structured to be disposed generally atop user's head 112 and adapted to be coupled to conduit 106 carrying the flow of breathing gas. Manifold portion 116 may be interchangeably referred to as manifold means 116. Manifold portion 116 is structured to be coupled to conduit 106, such as via an elbow 118 or any other suitable coupling member.

Further, tubing assembly 108 comprises a plurality of tubular portions 120, 122. Each tubular portion 120, 122 extends from manifold portion 116 to a distal end 124 which is structured to be coupled to patient interface device 110. Distal end 124 of each tubular portion 120, 122 is coupled to, and is in fluid communication with patient interface device 110.

Each tubular portion 120, 122 is structured to communicate the flow of breathing gas from manifold portion 116 to patient interface device 110. Further, manifold portion 116 provides fluid communication between conduit 106 and tubular portions 120, 122. As shown in the accompanying figures, when tubing assembly 108 is disposed on head 112 of user 102, manifold portion 116 is disposed generally at the top of head 112 of user 102 and tubular portions 120, 122 extend generally downward from manifold portion 116 to patient sealing element 114 of patient interface device 110.

In the illustrated embodiment of FIGS. 1A and 2, tubing assembly 108 comprises two tubular portions 120, 122. Each tubular portion 120, 122 defines a hollow passage (not shown) for allowing the flow of breathing gas therethrough. Tubular portions 120, 122 may be substantially similar in design. In the illustrated embodiment of FIGS. 1A and 2, tubular portions 120, 122 are symmetrical to each other with respect to manifold portion 116. Tubular portions 120, 122 are disposed at either side of manifold portion 116 such that tubular portions 120, 122 extend between manifold portion 116 and patient interface device 110. In the illustrated embodiment of FIGS. 1A and 2, tubular portions 120, 122 have an arcuate shape. In another exemplary embodiment, not shown, tubing assembly 108 may comprise a single tubular portion having a pair of arms resembling tubular portions 120, 122 that extend centrally, i.e., from manifold portion 116 generally over head 112 and nose, to patient sealing element 114.

As discussed above, tubular portions 120, 122 are embodied as hollow members for allowing the flow of breathing gas to pass therethrough. In various exemplary embodiments, tubular portions 120, 122 may have a generally non-circular cross-section, without any limitations. In an exemplary embodiment, tubular portions 120, 122 may have a generally D-shaped cross-section. As shown in FIG. 2, each tubular portion 120, 122 may define a patient facing portion 126 that comprises a generally flat side of the D-shape and is disposed adjacent head 112 (see FIG. 1A) of user 102 (see FIG. 1A). Further, a curved portion 127 of each tubular portion 120, 122 faces away from head 112 of user 102. In other exemplary embodiments, tubular portions 120, 122 may have any other cross-section. It should be noted that patient facing portion 126 may comprise a particular surface texture or may be formed from a separate material that presents a comfortable feel for user 102. Further, tubular portions 120, 122 may encircle, or partially encircle, head 112 of user 102. Accordingly, it is to be appreciated that tubing assembly 108, as a result of its basic structure and positioning, may not only direct the flow of breathing gas to patient interface device 110 (see FIG. 1A), but also generally functions as a frame that secures patient interface device 110 to head 112 of user 102.

In an exemplary embodiment, tubing assembly 108 may further comprise one or more pad members (not shown) that may be used to vary a feel, or to cushion the tubing assembly 108 on head 112 of user 102 and/or to better fit tubing assembly 108 to head 112 of user 102. The pad member may be formed from foam, gel, silicone, fabric plastic, or any other suitable material or combination of materials. In various exemplary embodiments, tubing assembly 108 may comprise one or more sections of removable cheek wraps (not shown), securable via suitable fasteners such as hook and loop (e.g., Velcro^{®} fasteners), or other fasteners. Cheek wraps may comprise fabric wraps that may be provided to customize a feel of tubing assembly 108 to a liking of a particular user.

Further, in order to help secure patient interface device 110 and tubing assembly 108 to head 112 of user 102, tubing assembly 108 may comprise a support assembly 128. Support assembly 128 may comprise a strap member 130 (shown in FIG. 1A) coupled to tubular portions 120, 122 which also encircles, or partially encircles, head 112 of user 102. That is, strap member 130 may be structured to engage a back of head 112 of user 102. In an exemplary embodiment of the present invention, strap member 130 may be structured to fit generally just below the occipital bone of user 102. Strap member 130 may comprise a design that may allow strap member 130 to naturally conform to the back of head 112 of user 102. One or more portions of strap member 130 may be formed from an elastic fabric (e.g., without limitation, spandex), but may also be formed from other fabrics, silicone, plastics, or any other suitable material or combination of materials such that portions of strap member 130 generally exhibits properties of an elastic strap that is typically easy to elongate but has recovery properties. It is to be appreciated that such exemplary materials are provided for example purposes only and that strap member 130 may be formed from other materials, without varying from the scope of the appended claims.

Strap member 130 may be coupled to each tubular portion 120, 122 by a mounting tab 132 disposed on each tubular portion 120, 122. Each mounting tab 132 may be fixed with respect to each tubular portion 120, 122. In such arrangements, each mounting tab 132 may be formed as an integral portion of each tubular portion 120, 122. In another exemplary embodiment, each mounting tab 132 may be formed separately therefrom and then rigidly coupled thereto. In other words, each mounting tab 132 may be rigidly coupled to a respective tubular portion 120, 122. In exemplary embodiments of the present invention, mounting tab 132 may be formed from silicone, however, it is to be appreciated that other suitable materials (e.g., without limitation, plastic, or fabric) may be employed, without varying from the scope of the appended claims.

In some exemplary embodiments, each mounting tab 132 may be adjustable with respect to tubing assembly 108 so as to allow for each mounting tab 132 to be adjusted to provide an optimal fit of tubing assembly 108 to head 112 of user 102. More particularly, each mounting tab 132 may be coupled to respective tubular portion 120, 122 in a manner such that each mounting tab 132 can generally slide along a portion of respective tubular portion 120, 122. In another exemplary embodiment, support assembly 128 may comprise a number of mounting tabs 132 placed along respective tubular portion 120, 122 to which strap member 130 may be selectively coupled by any suitable arrangement to adjust the placement of strap member 130. Alternatively, support assembly 128 may omit mounting tabs 132 such that strap member 130 may be generally placed anywhere along each tubular portion 120, 122. It is to be appreciated that support assembly 128 is provided for exemplary purposes only and that support assembly 128 of other design and/or construction may be employed, without varying from the scope of the appended claims.

Further, tubing assembly 108 comprises at least one adjustment unit 134, 136 configured to adjust a length of corresponding tubular portion 120, 122 from plurality of tubular portions 120, 122. Adjustment unit 134, 136 may be interchangeably referred to as adjustment means 134, 136. In the illustrated embodiment of FIG. 2, at least one adjustment unit 134, 136 comprises a pair of adjustment units 134, 136 disposed on opposing sides of manifold portion 116. Specifically, tubing assembly 108 comprises a first adjustment unit 134 and a second adjustment unit 136. First adjustment unit 134 is configured to adjust the length of tubular portion 120 and second adjustment unit 136 is configured to adjust the length of tubular portion 122. First and second adjustment units 134, 136 are similar in design. First and second adjustment units 134, 136 are symmetrical to each other with respect to manifold portion 116. In other exemplary embodiments, it may be contemplated that tubing assembly 108 may comprise a single adjustment unit or more than two adjustment units. In the illustrated embodiment of FIG. 2, first and second adjustment units 134, 136 are disposed proximal to manifold portion 116. However, adjustment units 134, 136 may be disposed at any other location along the length of corresponding tubular portion 120, 122.

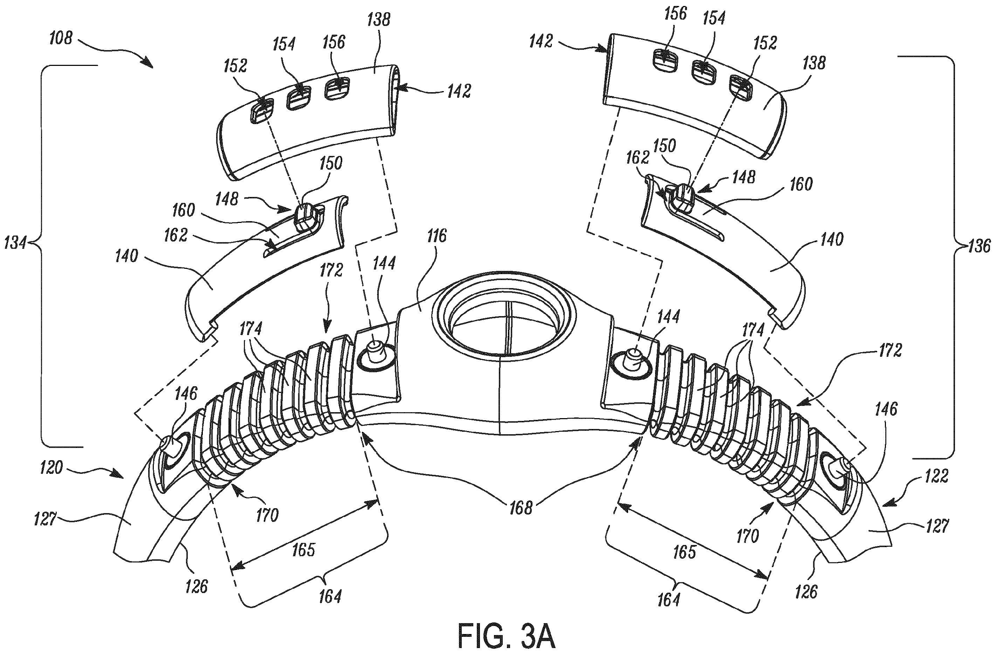

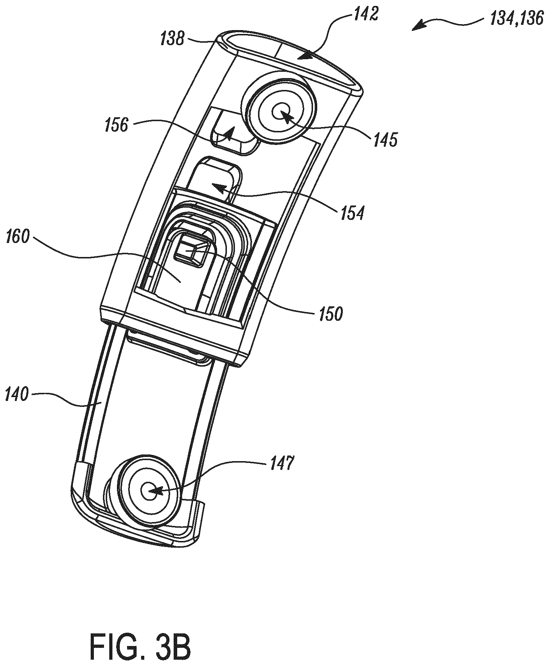

Further, at least one adjustment unit 134, 136 comprises a first adjustment member 138 integral with or fixedly connected to corresponding tubular portion 120, 122 and a second adjustment member 140 integral with or fixedly connected to corresponding tubular portion 120, 122. Each adjustment unit 134, 136 comprises one first adjustment member 138 and one second adjustment member 140. First and second adjustment members 138, 140 have a generally curved profile. One of first adjustment member 138 and second adjustment member 140 is disposed proximal to manifold portion 116, and the other one of first adjustment member 138 and second adjustment member 140 is disposed distal to manifold portion 116. In the illustrated embodiment of FIG. 2, first adjustment member 138 is disposed proximal to manifold portion 116, and second adjustment member 140 is disposed distal to manifold portion 116. Further, first and second adjustment members 138, 140 may have an arcuate shape that conforms with the shape of corresponding tubular portion 120, 122. In the illustrated embodiment of FIG. 2, each of first and second adjustment members 138, 140 is fixedly connected to corresponding tubular portion 120, 122. Further, first adjustment member 138 and second adjustment member 140 are movable relative to each other. Second adjustment member 140 may be at least partially received within a hollow portion 142 (shown in FIGS. 3A and 3B) defined by first adjustment member 138. Alternatively, first adjustment member 138 and second adjustment member 140 may be disposed such that first adjustment member 138 may be at least partially received within a hollow portion (not shown) defined by second adjustment member 140, without any limitations.

Referring to FIGS. 3A and 3B, in various exemplary embodiments, first and second adjustment members 138, 140 may be connected to corresponding tubular portion 120, 122 by a press fit, an interference fit, a snap fit, adhesives, and the like. In the illustrated embodiment of FIG. 3B, first and second adjustment members 138, 140 may comprise a first groove 145 and a second groove 147, respectively. Further, each tubular portion 120, 122 may comprise a first projecting tab 144 (see FIG. 3A) and a second projecting tab 146 (see FIG. 3A). First projecting tab 144 may align with first groove 145 in first adjustment member 138 for receipt of first projecting tab 144 within first groove 145 by a press fit. Further, second projecting tab 146 may align with second groove 147 in second adjustment member 140 for receipt of second projecting tab 146 within second groove 147 by a press fit. Alternatively, first and second adjustment members 138, 140 may be pivotally connected to corresponding tubular portion 120, 122. In other exemplary embodiments, any one of first and second adjustment members 138, 140 may be pivotally connected to corresponding tubular portion 120, 122. It should be noted that means for fixedly connecting first and second adjustment members 138, 140 to corresponding tubular portion 120, 122 as described herein is exemplary in nature and does not limit the scope of the appended claims.

Referring now to FIG. 3A, first adjustment member 138 and second adjustment member 140 are selectively and releasably locked to each other at a selected discrete position from a plurality of discrete positions. The plurality of discrete positions corresponds to a plurality of predetermined lengths of corresponding tubular portion 120, 122. The term "predetermined length" as used herein may refer to a variable length of tubular portions 120, 122 defined between manifold portion 116 and distal end 124. Specifically, the predetermined length may vary based on movement of first adjustment member 138 and second adjustment member 140. In some exemplary embodiments, the predetermined length may be defined as a curved length as tubular portions 120, 122 are arcuate in shape.

Further, the plurality of discrete positions may comprise an extended state, a contracted state, and one or more intermediate states between the extended state and the contracted state. In the illustrated embodiment of FIG. 3A, the plurality of discrete positions comprises three discrete positions. Although the exemplary embodiments described herein comprises three discrete positions, it should be noted that the plurality of discrete positions may comprise two discrete positions, for example, an extended state and a contracted state. In other exemplary embodiments, the plurality of discrete positions may comprise more than three discrete positions, for example, an extended state, a contracted state, and more than one intermediate states between the extended state and the contracted state, without any limitations. The plurality of discrete positions will be explained in detail later in this section.

Adjustment of tubing assembly 108 to different predetermined lengths may allow tubing assembly 108 to be used on various users having different head sizes. In some examples, each adjustment unit 134, 136 may allow the predetermined length of respective tubular portions 120, 122 to increase or decrease by a value between 10 mm and 40 mm. In some examples, each adjustment unit 134, 136 may allow the predetermined length of respective tubular portions 120, 122 to increase or decrease by a value between 10 mm and 20 mm. In one example, the predetermined length of each tubular portion 120, 122 may decrease by a value between 10 mm and 20 mm when tubing assembly 108 is switched from the extended state to the intermediate state. Similarly, the predetermined length of each tubular portion 120, 122 may increase by a value between 10 mm and 20 mm when tubing assembly 108 is switched from the intermediate state to the extended state. In one example, the predetermined length of each tubular portion 120, 122 may decrease by a value between 5 mm and 10 mm when tubing assembly 108 is switched from the extended state to the intermediate state. Similarly, the predetermined length of each tubular portion 120, 122 may increase by a value between 5 mm and 10 mm when tubing assembly 108 is switched from the intermediate state to the extended state.

In one example, the predetermined length of each tubular portion 120, 122 may decrease by a value between 10 mm and 20 mm when tubing assembly 108 is switched from the intermediate state to the contracted state. Similarly, the predetermined length of each tubular portion 120, 122 may increase by a value between 10 mm and 20 mm when tubing assembly 108 is switched from the contracted state to the intermediate state. In one example, the predetermined length of each tubular portion 120, 122 may decrease by a value between 5 mm and 10 mm when tubing assembly 108 is switched from the intermediate state to the contracted state. Similarly, the predetermined length of each tubular portion 120, 122 may increase by a value between 5 mm and 10 mm when tubing assembly 108 is switched from the contracted state to the intermediate state.

Tubing assembly 108 further comprises a locking means 148 for selectively and releasably locking adjustment unit 134, 136 at a selected discrete position from the plurality of discrete positions. Locking means 148 may comprise a pin 150 and a plurality of through apertures 152, 154, 156. It should be noted that locking means 148 described herein exemplary in nature and locking means 148 may comprise any other design or combination of components that together allow selective and releasable locking of adjustment units 134, 136 at the selected discrete position.

Specifically, first adjustment member 138 comprises plurality of through apertures 152, 154, 156 corresponding to the plurality of discrete positions. In the illustrated embodiment of FIG. 3A, first adjustment member 138 comprises three through apertures 152, 154, 156 corresponding to the three discrete positions. Through apertures 152, 154, 156 are equidistantly spaced apart from each other. Alternatively, first adjustment member 138 may comprise more than three through apertures 152, 154, 156 or less than three through apertures 152, 154, 156 based on a total number of discrete positions. Through apertures 152, 154, 156 have a substantially rectangular shape. Alternatively, through apertures 152, 154, 156 may have a square shape, a circular shape, an oval shape, a polygonal shape, and the like.

Further, second adjustment member 140 comprises pin 150 selectively, removably, and at least partially received within a selected through aperture 152, 154, 156 from plurality of through apertures 152, 154, 156 in order to releasably lock second adjustment member 140 to first adjustment member 138. In various exemplary embodiments, first adjustment member 138 may comprise a number of size indicators (not shown) corresponding to different discrete positions. The size indicators may indicate to user 102 regarding the discrete positions of tubing assembly 108. For example, a marking "L" adjacent to through aperture 152 may indicate that pin 150 should be received within through aperture 152 for tubing assembly 108 to fit users having a "large" head size. For example, the maximum predetermined length may be suitable for the "large" head size. Further, a marking "M" adjacent to through aperture 154 may indicate that pin 150 should be received within through aperture 154 for tubing assembly 108 to fit users having a "medium" head size. For example, the intermediate predetermined length may be suitable for the "medium" head size. Whereas a marking "S" adjacent to through aperture 156 may indicate that pin 150 should be received within through aperture 156 for tubing assembly 108 to fit users having a "small" head size. For example, the minimum predetermined length may be suitable for the "small" head size.

Further, a shape of pin 150 may be substantially similar to a shape of through apertures 152, 154, 156 so that pin 150 can be received within through apertures 152, 154, 156. In the illustrated embodiment of FIG. 3A, pin 150 is substantially rectangular in shape. Alternatively, pin 150 may comprise any other shape such as a substantially square shape, a substantially circular shape, a substantially oval shape, a substantially polygonal shape, and the like. Pin 150 is disposed distal from second groove 147 (see FIG. 3B) of second adjustment member 140. Further, pin 150 may be disposed at any other location on second adjustment member 140. Second adjustment member 140 may also comprise a biasing portion 160 attached to pin 150 and configured to resiliently bias pin 150 towards plurality of through apertures 152, 154, 156. Biasing portion 160 may be movable within a slot 162 of second adjustment member 140. Biasing portion 160 may bias second adjustment member 140 towards plurality of through apertures 152, 154, 156 such that pin 150 can be received within any one of through apertures 152, 154, 156.

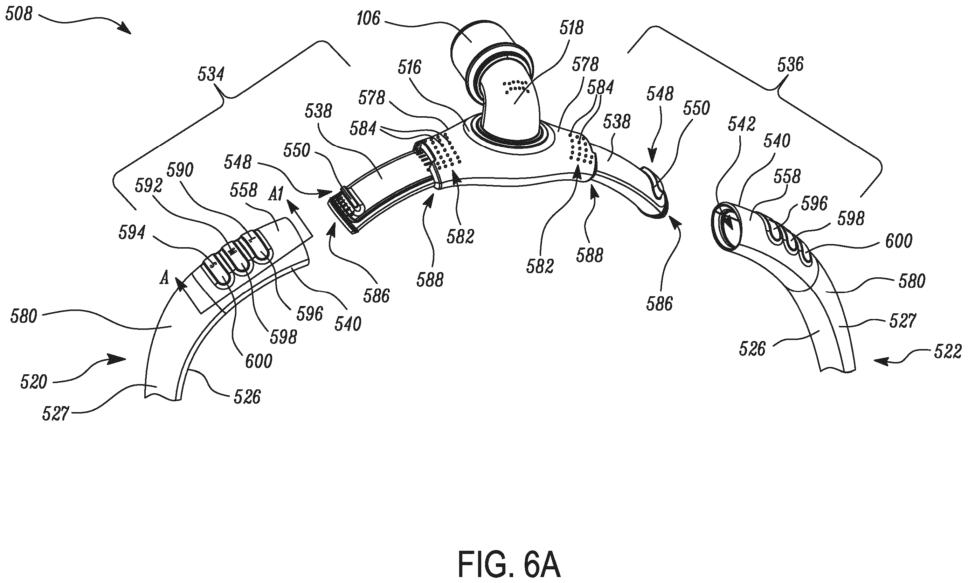

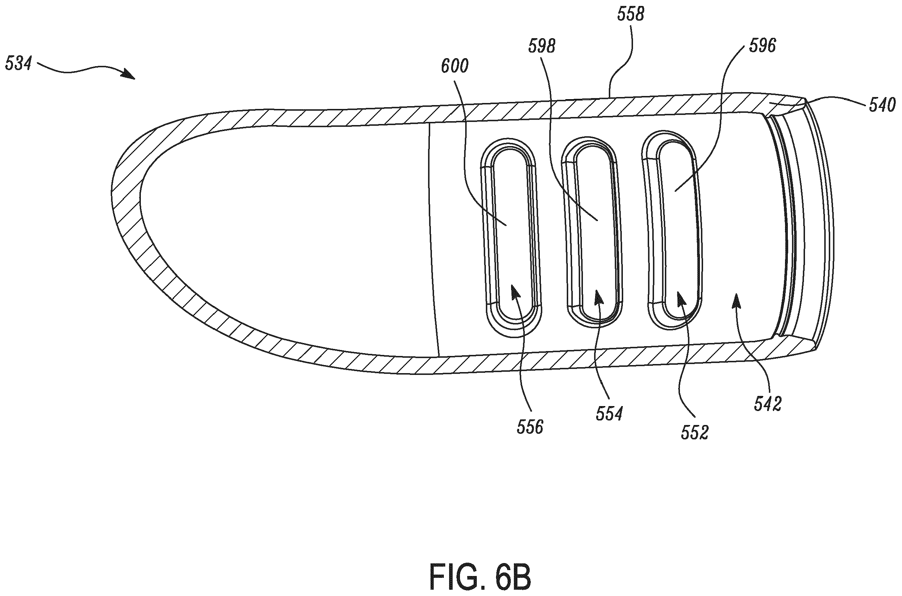

In alternate embodiments, second adjustment member 140 may comprise through apertures 152, 154, 156, whereas first adjustment member 138 may comprise pin 150, without any limitations. In an exemplary embodiment, through apertures 152, 154, 156 may be replaced by recesses (similar to recesses 552, 554, 556 shown in FIG. 6B) or blind apertures. In such an embodiment, first adjustment member 138 may comprise lugs (similar to a number of lugs 596, 598, 600 shown in FIG. 6A). In some other exemplary embodiments, first and second adjustment members 138, 140 may be releasably locked with each other using any other type of locking. For example, first and second adjustment members 138, 140 may be releasably locked with each other by a press fit, a snap fit, adhesives, or other fastening components such as snap fasteners, hook and loop fasteners, and the like, without any limitations.

Further, in various exemplary embodiments of the invention, at least one adjustment unit 134, 136 comprises an adjustable section 164 integral with corresponding tubular portion 120, 122. A relative movement between first adjustment member 138 and second adjustment member 140 causes adjustable section 164 to elongate or contract. Through such arrangement, the relative spacing between manifold portion 116 and patient interface device 110 may be adjusted in increments of the predetermined lengths without affecting the flow of breathing gas from corresponding tubular portion 120, 122 to patient interface device 110. It is to be appreciated that the predetermined lengths, as well as the general form of adjustable section 164 may be varied to meet requirements of a particular application, without varying from the scope of the appended claims.

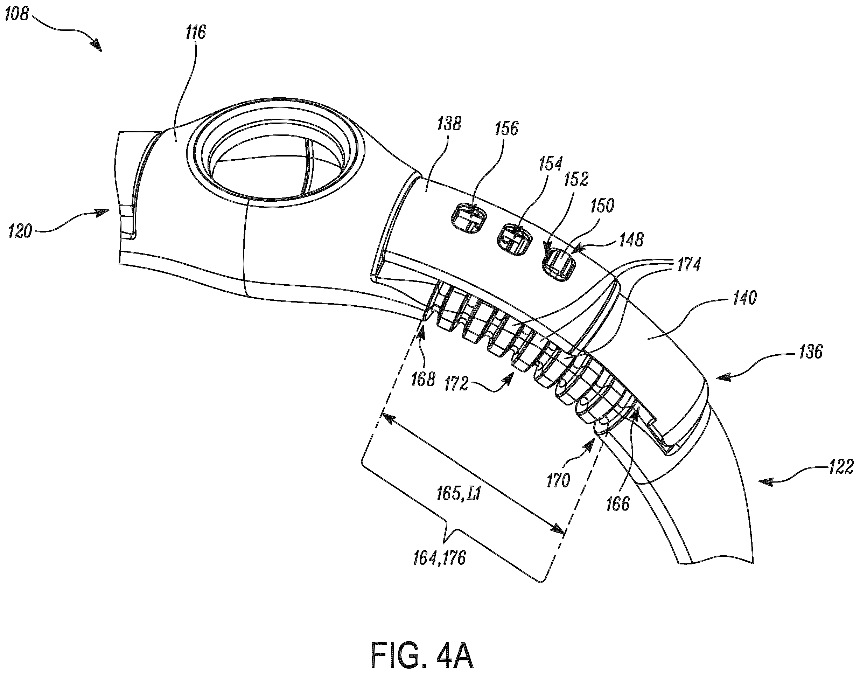

In various exemplary embodiments, each of first adjustment member 138 and second adjustment member 140 extends at least partially along a length 165 of adjustable section 164. Moreover, second adjustment member 140 may be spaced apart from adjustable section 164 and at least partially and slidably received within first adjustment member 138. More particularly, a gap 166 (shown in FIG. 4A) may be defined between second adjustment member 140 and adjustable section 164. Gap 166 may enable second adjustment member 140 to freely move without any interference from or engagement with adjustable section 164. In other exemplary embodiments, first adjustment member 138 may angularly move relative to second adjustment member 140, for example, when first adjustment member 138 is pivotally connected to corresponding tubular portion 120, 122. Further, a portion of adjustable section 164 may be slidably received within hollow portion 142 defined by first adjustment member 138.

Corresponding tubular portion 120, 122 and adjustable section 164 may comprise a similar material. In exemplary embodiments, tubular portion 120, 122 and adjustable section 164 may be made from plastic and/or silicone. Some parts of tubing assembly 108 may also be formed from other suitable materials (e.g., without limitation, fabric), without varying from the scope of the appended claims. In some exemplary embodiments, corresponding tubular portion 120, 122 and adjustable section 164 may be made from different materials. For example, corresponding tubular portion 120, 122 and adjustable section 164 may be made from materials having different durometer levels. Corresponding tubular portion 120, 122 and adjustable section 164 may be coupled together via a suitable process, or corresponding tubular portion 120, 122 may be integrally formed with adjustable section 164.

Adjustable section 164 comprises a first end 168 that is proximal to manifold portion 116 and a second end 170 opposite first end 168. First adjustment member 138 may be fixedly connected to corresponding tubular portion 120, 122 proximal to first end 168 of adjustable section 164. Specifically, first projecting tab 144 may be disposed proximal to first end 168. Further, second adjustment member 140 may be fixedly connected to corresponding tubular portion 120, 122 proximal to second end 170 of adjustable section 164 opposite to first end 168. Specifically, second projecting tab 146 may be disposed proximal to second end 170.

In the illustrated embodiment of FIG. 3A, adjustable section 164 is a corrugated section 172 integrally molded with corresponding tubular portion 120, 122 and configured to elongate or contract from a normal molded state of corrugated section 172. The normal molded state of corrugated section 172 may correspond to one of the extended state, the contracted state, and the one or more intermediate states. Further, first adjustment member 138 and second adjustment member 140 may be configured to selectively adjust corrugated section 172 to the other two of the extended state, the contracted state, and the one or more intermediate states.

Corrugated section 172 may comprise a bellows arrangement that elongates or contracts based on movement of corresponding first and second adjustment members 138, 140. Corrugated section 172 may comprise a number of corrugations 174 that elongates or contracts along length 165 of adjustable section 164. In the illustrated embodiment of FIG. 3A, corrugations 174 are spaced apart from each other. Specifically, when first adjustment member 138 and second adjustment member 140 are moved, spacing between two adjacently disposed corrugations 174 may change to allow elongation or contraction of corrugated section 172. In various other exemplary embodiments, corrugated section 172 may comprise a continuous helical or spiral design. In the illustrated embodiment of FIG. 3A, adjustable section 164 of each tubular portion 120, 122 comprises a single corrugated section 172 having corrugations 174. Alternatively, adjustable section 164 of each tubular portion 120, 122 may comprise multiple corrugated sections (similar to corrugated section 172) that may be spaced apart from each other along length 165 of adjustable section 164, such that each of the multiple corrugated sections comprises a number of corrugations, without any limitations.

In the illustrated embodiment of FIG. 3A, each corrugation 174 has a similar shape and size. Further, corrugations 174 comprise a generally oval cross-section. Alternatively, corrugations 174 may comprise any other shape, such as a rectangular cross-section, a square cross-section, a circular cross-section, a flared cross-section, and the like. In an exemplary embodiment, corrugations 174 may be integrally formed with corresponding tubular portion 120, 122. In such embodiments, corrugations 174 may be made of a material that is similar to the material of tubular portions 120, 122. In alternate embodiments, corrugations 174 may be manufactured separately and subsequently assembled with corresponding tubular portion 120, 122. In such embodiments, corrugations 174 may be made of a material that may be different from the material of tubular portions 120, 122. Further, a thickness of each corrugation 174 may be chosen such that corrugations 174 can endure a manufacturing process and may accommodate realistic variations in tolerances. It should be noted that the present invention is not limited by a number of corrugations 174, the shape of corrugations 174, dimensions of corrugations 174, and the material of corrugations 174.

It should be further noted that a design of adjustable section 164 described herein is exemplary in nature, and adjustable section 164 may comprise any other design provided that adjustable section 164 elongates or contracts based on relative movement between first adjustment member 138 and second adjustment member 140. In various exemplary embodiments, adjustable section 164 may comprise another design. For example, adjustable section 164 may comprise a combination of one or more continuous helical members disposed around a hollow stretchable fibrous material that may allow the flow of breathing gas therethrough, without any limitations.

In various exemplary embodiments, adjustment units 134, 136 may comprise any other design or combination of components to dispose adjustment units 134, 136 in various discrete positions. For example, adjustment units 134, 136 may comprise a single flexible strap or a pair of flexible straps. In various exemplary embodiments, adjustment units 134, 136 may comprise a strap and buckle arrangement, or a pair of straps that can be removably connected to each other by techniques such as, hook and loop fasteners (e.g., Velcro^{®} fasteners), snap fasteners, and the like.

In various exemplary embodiments, tubular portions 120, 122 may comprise one or more additional flexible sections that are integral with corresponding tubular portion 120, 122. For example, such flexible sections may be present between distal end 124 and corresponding adjustment unit 134, 136. The flexible sections may be similar to corrugated section 172, or the flexible sections may comprise any other design, without any limitations.

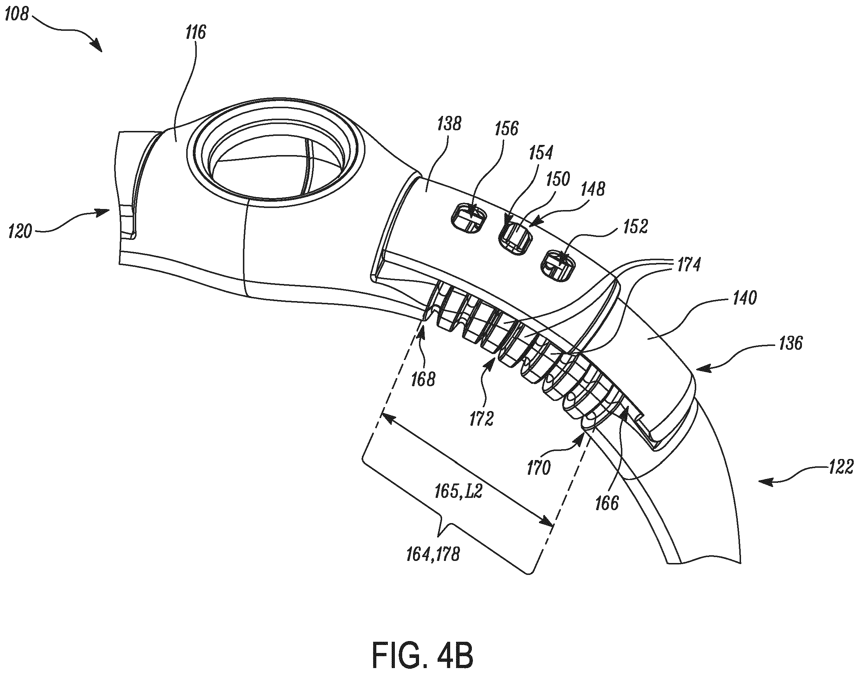

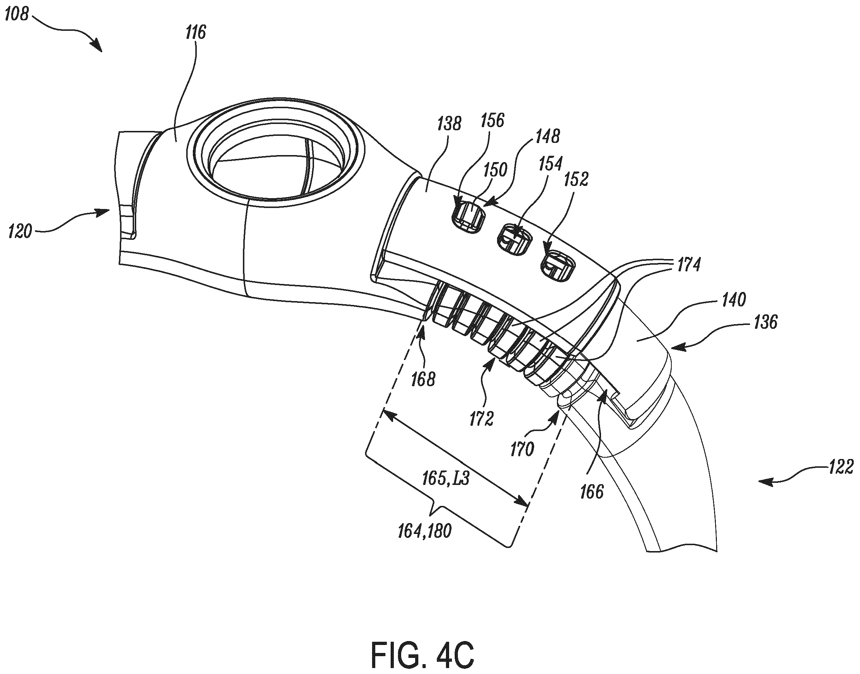

Various discrete positions associated with tubing assembly 108 will now be explained with reference to FIGS. 4A, 4B, and 4C. FIG. 4A illustrates first adjustment member 138 and second adjustment member 140 selectively and releasably locked to each other at the extended state. Specifically, adjustable section 164 is in an extended position 176. For explanatory purposes, only tubular portion 122 and adjustment unit 136 are explained in detail with reference to FIG. 4A. However, it should be noted that details provided herein are equally applicable to tubular portion 120 and adjustment unit 134. Extended position 176 may correspond to the maximum predetermined length such that tubing assembly 108 is usable by user 102 (see FIG. 1A) having the "large" head size. As illustrated in Fig. 4A, in extended position 176, adjustable length 165 of adjustable section 164 corresponds to a maximum length "L1". In extended position 176, pin 150 is received within through aperture 152. In one exemplary embodiment, the normal molded state may correspond to extended position 176 such that adjustable section 164 can contract, for example, up to two sizes, i.e., to an intermediate position 178 (shown in FIG. 4B) and a contracted position 180 (shown in FIG. 4C).

As per application requirements, if adjustable section 164 is to be disposed in intermediate position 178 or contracted position 180, pin 150 may be disengaged from through aperture 152. Further, first and second adjustment members 138, 140 are moved towards each other causing corrugated section 172 to contract. Moreover, pin 150 may be engaged with through apertures 154 or 156 for disposing adjustable section 164 in intermediate position 178 or contracted position 180, respectively. It should be noted that when tubing assembly 108 switches to intermediate position 178 from extended position 176, adjustable length 165 may decrease from maximum length "L1" by a value between 5 mm and 10 mm, or a value between 10 mm and 20 mm. Similarly, when tubing assembly 108 switches to contracted position 180 from extended position 176, adjustable length 165 may decrease from maximum length "L1" by a value between 10 mm and 20 mm, or a value between 20 mm and 40 mm.

FIG. 4B illustrates first adjustment member 138 and second adjustment member 140 selectively and releasably locked to each other at the intermediate state. Specifically, adjustable section 164 is in intermediate position 178. For explanatory purposes, only tubular portion 122 and adjustment unit 136 are explained in detail with reference to FIG. 4B. However, it should be noted that details provided herein are equally applicable to tubular portion 120 and adjustment unit 134. Intermediate position 178 may correspond to the intermediate predetermined length, that is smaller than the maximum predetermined length, such that tubing assembly 108 is usable by user 102 (see FIG. 1A) having the "medium" head size. As illustrated in Fig. 4B, in intermediate position 178, adjustable length 165 of adjustable section 164 corresponds to an intermediate length "L2". In intermediate position 178, pin 150 is received within through aperture 154. In one exemplary embodiment, the normal molded state may correspond to intermediate position 178 such that adjustable section 164 can elongate or contract, for example, by one size from intermediate position 178.

As per application requirements, if adjustable section 164 is to be disposed in extended position 176 (shown in FIG. 4A) or contracted position 180 (shown in FIG. 4C), pin 150 may be disengaged from through aperture 154. Further, for disposing adjustable section 164 in extended position 176, first and second adjustment members 138, 140 are moved away from each other causing corrugated section 172 to elongate. Moreover, pin 150 is engaged with through aperture 152 for disposing adjustable section 164 in extended position 176. For disposing adjustable section 164 in contracted position 180, first and second adjustment members 138, 140 are moved towards each other causing corrugated section 172 to contract. Moreover, pin 150 may be engaged with through aperture 156 for disposing adjustable section 164 in contracted position 180. It should be noted that when tubing assembly 108 switches to extended position 174 from intermediate position 178, adjustable length 165 may increase from intermediate length "L2" by a value between 5 mm and 10 mm, or a value between 10 mm and 20 mm. Similarly, when tubing assembly 108 switches to contracted position 180 from intermediate position 178, adjustable length 165 may decrease from intermediate length "L2" by a value between 5 mm and 10 mm, or a value between 10 mm and 20 mm.

FIG. 4C illustrates first adjustment member 138 and second adjustment member 140 selectively and releasably locked to each other at the contracted state. Specifically, adjustable section 164 is in contracted position 180. For explanatory purposes, only tubular portion 122 and adjustment unit 136 are explained in detail with reference to FIG. 4C. However, it should be noted that details provided herein are equally applicable to tubular portion 120 and adjustment unit 134. Contracted position 180 may correspond to the minimum predetermined length, that is smaller than the intermediate predetermined length, such that tubing assembly 108 is usable by user 102 (see FIG. 1A) having the "small" head size. As illustrated in Fig. 4C, in contracted position 180, adjustable length 165 of adjustable section 164 corresponds to a minimum length "L3". In contracted position 180, pin 150 is received within through aperture 156. In one exemplary embodiment, the normal molded state may correspond to contracted position 180 such that adjustable section 164 can elongate, for example, up to two sizes, i.e., to intermediate position 178 (shown in FIG. 4B) and extended position 176 (shown in FIG. 4A).

As per application requirements, if adjustable section 164 is to be disposed in intermediate position 178 or extended position 176, pin 150 is disengaged from through aperture 156. Further, first and second adjustment members 138, 140 are moved away from each other causing corrugated section 172 to elongate. Moreover, pin 150 may be engaged with through apertures 152 or 154 for disposing adjustable section 164 in extended position 176 or intermediate position 178, respectively. It should be noted that when tubing assembly 108 switches to intermediate position 178 from contracted position 180, adjustable length 165 may increase from minimum length "L3" by a value between 5 mm and 10 mm, or a value between 10 mm and 20 mm. Similarly, when tubing assembly 108 switches to extended position 174 from contracted position 180, adjustable length 165 may increase from minimum length "L3" by a value between 10 mm and 20 mm, or a value between 20 mm and 40 mm.

Referring to FIGS. 4A to 4C, in an example, a difference between maximum length "L1" and intermediate length "L2" is between 5 mm and 10 mm. In another example, the difference between maximum length "L1" and intermediate length "L2" is between 10 mm and 20 mm. In an example, a difference between intermediate length "L2" and minimum length "L3" is between 5 mm and 10 mm. In another example, the difference between intermediate length "L2" and minimum length "L3" is between 10 mm and 20 mm. In an example, a difference between maximum length "L1" and minimum length "L3" is between 10 mm and 20 mm. In an example, the difference between maximum length "L1" and minimum length "L3" is between 20 mm and 40 mm. Since each tubular portion 120, 122 includes respective adjustable unit 134, 136, an overall length of tubing assembly 108 can be adjusted by a total adjustment length that is about twice a difference between maximum length "L1" and minimum length "L3". In an example, the total adjustment length of tubing assembly 108 is between 20 mm and 40 mm. In another example, the total adjustment length of tubing assembly 108 is between 40 mm and 80 mm. In some examples, the total adjustment length of tubing assembly 108 is about 30 mm or about 75 mm.

Tubing assembly 108 may be manufactured using an injection molding process. Further, tubing assembly 108 may be formed as a single unitary member. Such a single unitary tubing assembly 108 having uniform material throughout may be simple to manufacture and may be cost effective. Alternatively, tubing assembly 108 may be formed from a number of separately formed components that are coupled together via a suitable process. For example, first and second adjustment members 138, 140 may be manufactured as separate components that may be fixedly connected to corresponding tubular portion 120, 122

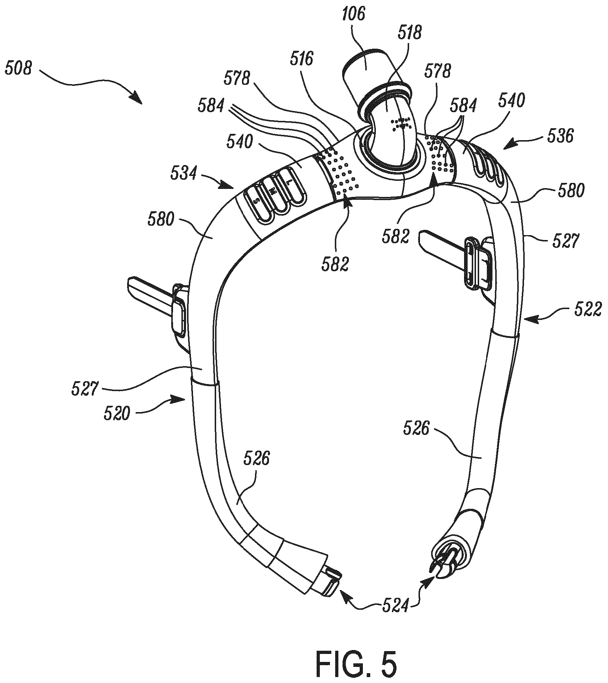

FIGS. 5 and 6A illustrate another design of a tubing assembly 508 for use with patient interface device 110 (see FIG. 1A) in delivering the flow of breathing gas to the airway of user 102 (see FIG. 1A). Tubing assembly 508 may be interchangeably referred to as tubing means 508. With reference to FIG. 5, tubing assembly 508 comprises a manifold portion 516 structured to be disposed generally atop user's head 112 (see FIG. 1A) and adapted to be coupled to conduit 106 (see FIG. 1A) carrying the flow of breathing gas. Manifold portion 516 may be interchangeably referred to as manifold means 516. Manifold portion 516 is structured to be coupled to conduit 106, such as via an elbow 518 having a tapered design or other suitable coupling member. Further, elbow 518 illustrated herein exhibits an anti-asphyxia feature.

Tubing assembly 508 further comprises a plurality of tubular portions 520, 522. Each tubular portion 520, 522 extends from manifold portion 516 to a distal end 524 which is structured to be coupled to patient interface device 110. Distal end 524 of tubular portion 520, 522 is coupled to, and is in fluid communication with, patient interface device 110. Further, each tubular portion 520, 522 may be structured to communicate the flow of breathing gas from manifold portion 516 to patient interface device 110. Specifically, manifold portion 516 provides fluid communication between conduit 106 and tubular portions 520, 522. When tubing assembly 508 is disposed on head 112 of user 102, manifold portion 516 is disposed generally at the top of head 112 of user 102 and tubular portions 520, 522 extend generally downward from manifold portion 516 to patient sealing element 114 (see FIG. 1A) of patient interface device 110. In the illustrated embodiment of FIGS. 5 and 6A, tubing assembly 508 comprises two tubular portions 520, 522.

Each of tubular portions 520, 522 comprise a hollow passage (not shown) for allowing the flow of breathing gas therethrough. Tubular portions 520, 522 are similar in design. Tubular portions 520, 522 are symmetrical to each other with respect to manifold portion 516. Tubular portions 520, 522 are disposed at either sides of manifold portion 516 such that tubular portions 520, 522 extend between manifold portion 516 and patient interface device 110. In the illustrated embodiment of FIGS. 5 and 6A, tubular portions 520, 522 have an arcuate shape. In another exemplary embodiment, not shown, tubing assembly 508 may comprise a single tubular portion having a pair of arms resembling tubular portions 520, 522 that extend centrally, i.e., from manifold portion 516 generally over head 112 and nose, to patient sealing element 114.

As discussed above, tubular portions 520, 522 are embodied as hollow members for allowing the flow of breathing gas to pass therethrough. In various exemplary embodiments, tubular portions 520, 522 may have a generally non-circular cross-section, without any limitations. In an exemplary embodiment, tubular portions 520, 522 may have a generally D-shaped cross-section. Further, each tubular portions 520, 522 may define a patient facing portion 526 that comprises a generally flat side of the D-shape and is disposed adjacent head 112. Further, a curved portion 527 of tubular portion 520, 522 faces away from head 112. In other exemplary embodiments, tubular portions 520, 522 may have any other cross-section. It should be noted that patient facing portion 526 may comprise a particular surface texture or may be formed from a separate material that presents a comfortable feel for user 102. Further, tubular portions 520, 522 may encircle, or partially encircle, head 112 of user 102. Accordingly, it is to be appreciated that tubing assembly 508, as a result of its basic structure and positioning, may not only direct the flow of breathing gas to patient interface device 110, but also generally functions as a frame that secures patient interface device 110 to head 112 of user 102.

In an exemplary embodiment, tubing assembly 508 may further comprise one or more pad members (not shown) that may be used to vary a feel of tubing assembly 508 on head 112 of user 102 and/or to better fit tubing assembly 508 to head 112 of user 102. In various exemplary embodiments, tubing assembly 508 may comprise one or more sections of removable cheek wraps (not shown), securable via suitable fasteners such as hook and loop (e.g., Velcro^{®} fasteners) or other fasteners.

Further, in order to help secure patient interface device 110 and tubing assembly 508 to head 112 of user 102, tubing assembly 508 may further comprise a support assembly (not shown). The support assembly may be similar to support assembly 128 described with reference to FIG. 2. The support assembly may comprise a strap member that may be coupled to each tubular portion 520, 522 by a mounting tab disposed on each tubular portion 520, 522. In other embodiments, tubing assembly 508 may omit the support assembly.

Further, a corresponding tubular portion 520, 522 comprises a first tubular section 578 and a second tubular section 580 separate from first tubular section 578. First and second tubular sections 578, 580 may together define the hollow passage for passage of the flow of the breathing gas therethrough. First tubular section 578 may comprise a stepped design. First tubular section 578 may extend from manifold portion 516 and second tubular section 580 may comprise distal end 524. Further, first tubular section 578 may comprise a gripping region 582 disposed adjacent to a first adjustment member 538 (shown in FIG. 6A). Gripping region 582 may comprise a plurality of dimples 584. Dimples 584 may provide an improved gripping surface for user 102 while switching between different discrete positions. Moreover, second tubular section 580 may be distal from manifold portion 516 such that second tubular section 580 connects to patient interface device 110.

Further, tubing assembly 508 comprises at least one adjustment unit 534, 536 configured to adjust a length of corresponding tubular portion 520, 522 from plurality of tubular portions 520, 522. Adjustment unit 534, 536 may be interchangeably referred to as adjustment means 534, 536. In the illustrated embodiment of FIGS. 5 and 6A, at least one adjustment unit 534, 536 comprises a pair of adjustment units 534, 536 disposed on opposing sides of manifold portion 516. Specifically, tubing assembly 508 may comprise a first adjustment unit 534 and a second adjustment unit 536. First adjustment unit 5134 is configured to adjust the length of tubular portion 520 and second adjustment unit 536 is configured to adjust the length of tubular portion 522. First and second adjustment units 534, 536 are similar in design. First and second adjustment units 534, 536 are symmetrical to each other with respect to manifold portion 516. In other exemplary embodiments, it may be contemplated that tubing assembly 508 may comprise a single adjustment unit or more than two adjustment units. In the illustrated embodiment of FIGS. 5 and 6A, adjustment units 534, 536 are disposed proximal to manifold portion 516. However, adjustment units 534, 536 may be disposed at any other location along the length of corresponding tubular portion 520, 522.

As shown in FIG. 6A, at least one adjustment unit 534, 536 comprises first adjustment member 538 integral with or fixedly connected to corresponding tubular portion 520, 522 and a second adjustment member 540 integral with or fixedly connected to corresponding tubular portion 520, 522. Each adjustment unit 534, 536 comprises one first adjustment member 538 and one second adjustment member 540. One of first adjustment member 538 and second adjustment member 540 is disposed proximal to manifold portion 516, and the other one of first adjustment member 538 and second adjustment member 540 is disposed distal to manifold portion 516. In the illustrated embodiment of FIG. 6A, first adjustment member 538 is disposed proximal to manifold portion 516, and second adjustment member 540 is disposed distal to manifold portion 516. Further, in the illustrated embodiment of FIG. 6A, first adjustment member 538 is fixedly connected to first tubular section 578 and second adjustment member 540 is fixedly connected to second tubular section 580. In other exemplary embodiments, first adjustment member 538 and second adjustment member 540 may be removably connected to first and second tubular sections 578, 580, respectively, by a snap fit, a press fit, adhesives, and the like.

Further, first adjustment member 538 and second adjustment member 540 are selectively and releasably locked to each other at a selected discrete position from a plurality of discrete positions. The plurality of discrete positions corresponds to a plurality of predetermined lengths of corresponding tubular portion 520, 522.

Further, the plurality of discrete positions may comprise an extended state, a contracted state, and one or more intermediate states between the extended state and the contracted state. In the illustrated embodiment of FIG. 6A, the plurality of discrete positions comprises three discrete positions. Although the exemplary embodiments described herein comprises three discrete positions, it should be noted that the plurality of discrete positions may comprise two discrete positions, for example, an extended state and a contracted state. In other exemplary embodiments, the plurality of discrete positions may comprise more than three discrete positions, for example, an extended state, a contracted state, and more than one intermediate states between the extended state and the contracted state, without any limitations. The plurality of discrete positions will be explained in detail later in this section.

Adjustment of tubing assembly 508 to different predetermined lengths may allow tubing assembly 508 to be used on various users having different head sizes. In some examples, each adjustment unit 534, 536 may allow the predetermined length of respective tubular portions 520, 522 to increase or decrease by a value between 10 mm and 40 mm. In some examples, each adjustment unit 534, 536 may allow the predetermined length of respective tubular portions 520, 522 to increase or decrease by a value between 10 mm and 20 mm. In one example, the predetermined length of each tubular portion 520, 522 may decrease by a value between 10 mm and 20 mm when tubing assembly 508 is switched from the extended state to the intermediate state. Similarly, the predetermined length of each tubular portion 520, 522 may increase by a value between 10 mm and 20 mm when tubing assembly 508 is switched from the intermediate state to the extended state. In one example, the predetermined length of each tubular portion 520, 522 may decrease by a value between 5 mm and 10 mm when tubing assembly 508 is switched from the extended state to the intermediate state. Similarly, the predetermined length of each tubular portion 520, 522 may increase by a value between 5 mm and 10 mm when tubing assembly 508 is switched from the intermediate state to the extended state.

In one example, the predetermined length of each tubular portion 520, 522 may decrease by a value between 10 mm and 20 mm when tubing assembly 508 is switched from the intermediate state to the contracted state. Similarly, the predetermined length of each tubular portion 520, 522 may increase by a value between 10 mm and 20 mm when tubing assembly 508 is switched from the contracted state to the intermediate state. In one example, the predetermined length of each tubular portion 520, 522 may decrease by a value between 5 mm and 10 mm when tubing assembly 508 is switched from the intermediate state to the contracted state. Similarly, the predetermined length of each tubular portion 520, 522 may increase by a value between 5 mm and 10 mm when tubing assembly 508 is switched from the contracted state to the intermediate state.

Further, tubing assembly 508 comprises a locking means 548 for selectively and releasably locking adjustment unit 534, 536 at a selected discrete position from the plurality of discrete positions. Locking means 548 may comprise a protrusion 550 and a plurality of recesses 552, 554, 556 (shown in FIG. 6B).

First adjustment member 538 comprises an end 586 proximal to second tubular section 580 and an end 588 proximal to first tubular section 578. In the illustrated embodiment of FIG. 6A, first adjustment member 538 is integral with first tubular section 578 and comprises protrusion 550 proximal to end 586 thereof. Dimensions of first tubular section 578 may be slightly greater than dimensions of first adjustment member 538. Although a single protrusion 550 is illustrated herein, first adjustment member 538 may comprise more than one protrusion. In various exemplary embodiments, first adjustment member 538 may comprise three protrusions. Further, protrusion 550 may be disposed at any location on first adjustment member 538. In the illustrated embodiment of FIG. 6A, protrusion 550 is substantially oval in shape. Alternatively, protrusion 550 may comprise any other shape, such as a substantially square shape, a substantially circular shape, a substantially rectangular shape, a substantially polygonal shape, and the like. In one exemplary embodiment, first tubular section 578 comprises a first material and first adjustment member 538 comprises a second material different from the first material. In various exemplary embodiments, the first material comprises silicone and the second material comprises polycarbonate. Alternatively, first tubular section 578 and first adjustment member 538 may be made of any other material, without any limitations.

Further, first adjustment member 538 and second adjustment member 540 are movable relative to each other. First adjustment member 538 is configured to be at least partially and slidably received within second adjustment member 540. Specifically, first adjustment member 538 may be at least partially received within a hollow portion 542 defined by second adjustment member 540. Accordingly, dimensions of second adjustment member 540 may be slightly greater than dimensions of first adjustment member 538 to allow receipt of first adjustment member 538 within second adjustment member 540. Alternatively, first adjustment member 538 and second adjustment member 540 may be disposed such that second adjustment member 540 may be at least partially received within a hollow portion (not shown) defined by first adjustment member 538, without any limitations.

Further, second adjustment member 540 is integral with second tubular section 580 and comprises plurality of recesses 552, 554, 556 corresponding to the plurality of discrete positions. Specifically, second adjustment member 540 comprises lugs 596, 598, 600 that project outwards from an outer surface 558 of second adjustment member 540. As shown in FIG. 6B, lugs 596, 598, 600 define respective recesses 552, 554, 556, such that recesses 552, 554, 556 face the hollow passage through which the breathing gas flows. Recesses 552, 554, 556 are equidistantly spaced apart from each other. Alternatively, recesses 552, 554, 556 may not be equidistantly spaced apart from each other and a placement of recesses 552, 554, 556 may vary based on a desired discrete position, without any limitations. Plurality of recesses 552, 554, 556 are configured to selectively, removably, and at least partially receive protrusion 550 (see FIG. 6A) of first adjustment member 538 (see FIG. 6A) therein in order to releasably lock first adjustment member 538 to second adjustment member 540. Specifically, in response to sliding of first adjustment member 538 within second adjustment member 540, first and/or second adjustment member 538, 540 may be configured to deform in order to allow protrusion 550 to be selectively inserted into or removed from plurality of recesses 552, 554, 556.

In the illustrated embodiment of FIG. 6B, second adjustment member 540 comprises three recesses 552, 554, 556 corresponding to the three discrete positions. Alternatively, second adjustment member 540 may comprise more than three recesses, or less than three recesses based on a total number of the discrete positions. Further, a shape of recesses 552, 554, 556 may be substantially similar to a shape of protrusion 550 so that protrusion 550 can be received within recesses 552, 554, 556. In the illustrated embodiment of FIG. 6A, recesses 552, 554, 556 have an oval shape. Alternatively, recesses 552, 554, 556 may have a substantially square shape, a substantially circular shape, a substantially rectangular shape, a substantially polygonal shape, and the like. In other exemplary embodiments, when first adjustment member 538 at least partially receives second adjustment member 540, first adjustment member 538 may comprise recesses 552, 554, 556, whereas second adjustment member 540 may comprise protrusion 550, without any limitations.

In an exemplary embodiment, recesses 552, 554, 556 may be replaced by through apertures (similar to through apertures 152, 154, 156 shown in FIG. 3A). In such an embodiment, second adjustment member 540 may omit lugs 596, 598, 600. In other exemplary embodiments, first and second adjustment members 538, 540 may be releasably locked with each other using any other means. For example, first and second adjustment members 538, 540 may be releasably locked with each other by a press fit, a snap fit, a biasing mechanism, adhesives, or other fastening components such as snap fasteners, hook and loop fasteners, and the like, without any limitations.

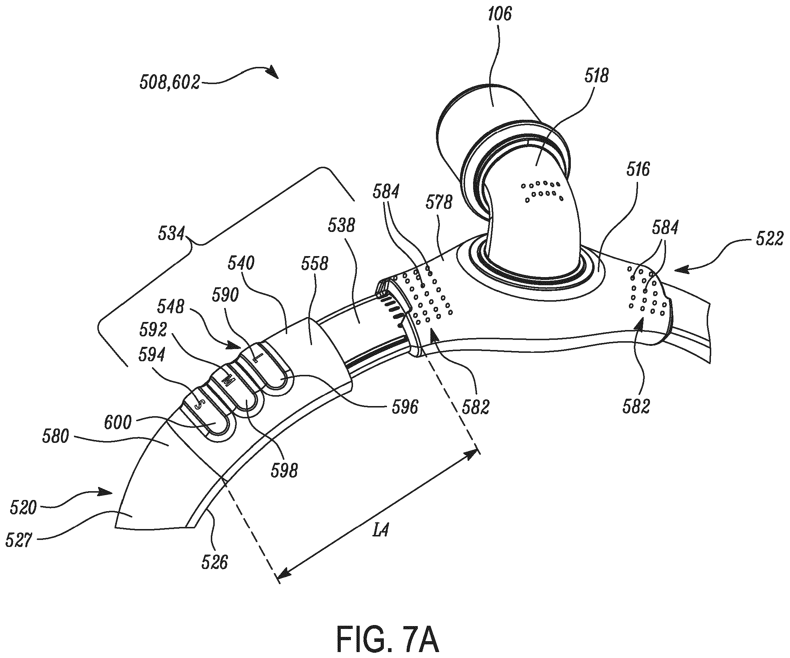

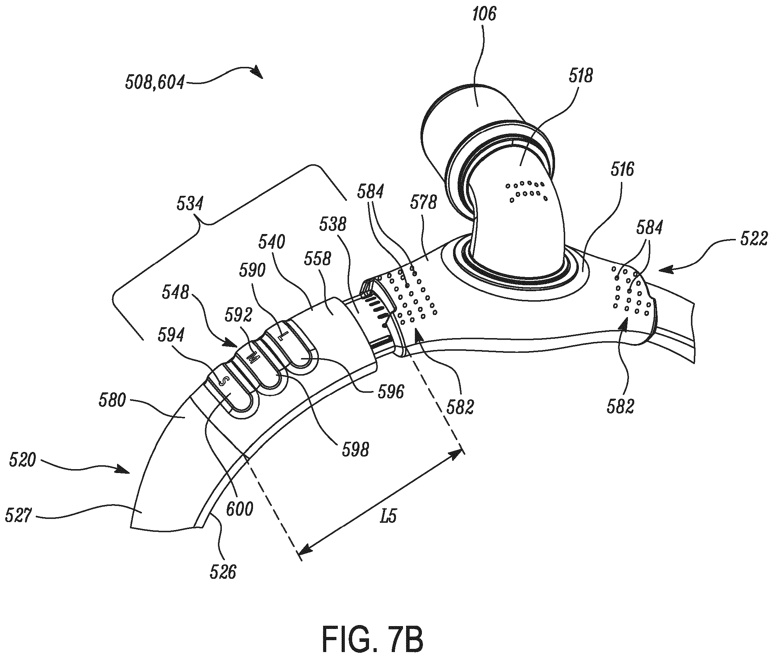

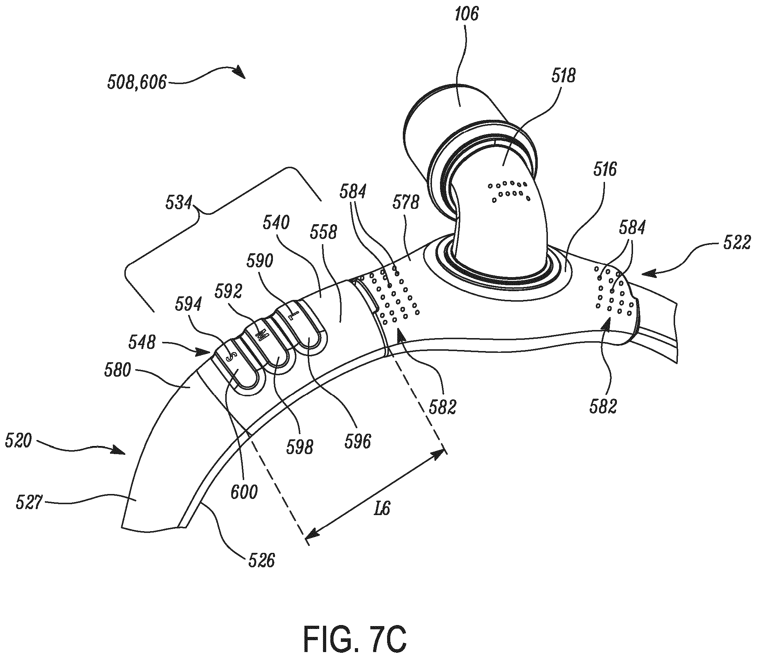

Referring again to FIG. 6A, second adjustment member 540 may also comprise a number of size indicators 590, 592, 594 corresponding to different discrete positions. In the illustrated embodiment of FIG. 6A, second adjustment member 540 comprises size indicator 590 having a marking "L" which is an abbreviation for large size, such that when protrusion 550 is received within recess 552 (see FIG. 6B), size indicator 590 having marking "L" indicates to user 102 (see FIG. 1A) that tubing assembly 508 is in an extended position 602 (shown in FIG. 7A). Further, second adjustment member 540 comprises size indicator 592 having a marking "M" which is an abbreviation for medium size, such that when protrusion 550 is received within recess 554 (see FIG. 6B), size indicator 592 having marking "M" indicates to user 102 that tubing assembly 508 is in an intermediate position 604 (shown in FIG. 7B). Moreover, second adjustment member 540 comprises size indicator 594 having a marking "S" which is an abbreviation for small size, such that when protrusion 550 is received within recess 556 (see FIG. 6B), size indicator 594 having marking "S" indicates to user 102 that tubing assembly 508 is in a contracted position 606 (shown in FIG. 7C). It should be noted that markings used for size indicators 590, 592, 594 are exemplary in nature, and size indicators 590, 592, 594 may comprise any other type of marking, without any limitations. In other embodiments, size indicators 590, 592, 594 may be provided on first adjustment member 538 to provide user 102 with visual access regarding a current position of tubing assembly 508. In such an embodiment, size indicators 590, 592, 594 may be marked on first adjustment member 538 such that size indicators 590, 592, 594 may be revealed to user 102 based on switching between the discrete positions for notifying user 102 regarding a current position of tubing assembly 508.

In one exemplary embodiment, second tubular section 580 and second adjustment member 540 are made of a similar material. In an exemplary embodiment, second adjustment member 540 may be made of a deformable material. In various exemplary embodiments, second adjustment member 540 may comprise a transparent or translucent material to provide visual access to user 102 regarding a current position of tubing assembly 508. In other embodiments, lugs 596, 598, 600 may be made of a transparent or translucent material. In various exemplary embodiments, each of second tubular section 580 and second adjustment member 540 comprises silicone. In various exemplary embodiments, tubular portions 520, 522 may comprise one or more additional flexible sections that are integral with corresponding tubular portion 520, 522. For example, such flexible sections may be present between distal end 524 (see FIG. 5) and corresponding adjustment unit 534, 536. The flexible sections may be similar to corrugated section 172 explained with reference to FIG. 3A, or the flexible sections may comprise any other design, without any limitations.

Various discrete positions associated with tubing assembly 508 will now be explained with reference to FIGS. 7A, 7B, and 7C. FIG. 7A illustrates tubing assembly 508 in extended position 602. For explanatory purposes, only tubular portion 520 and adjustment unit 534 are explained in detail with reference to FIG. 7A. However, it should be noted that details provided herein are equally applicable to tubular portion 522 and adjustment unit 536. Extended position 602 may correspond to the maximum predetermined length such that tubing assembly 508 is usable by user 102 (see FIG. 1A) having a "large" head size. As illustrated in Fig. 7A, in extended position 602, adjustable unit 536 has a maximum length "L4". In extended position 602, protrusion 550 (see FIG. 6A) is received within recess 552 (see FIG. 6B). In one exemplary embodiment, tubing assembly 508 can slide up to two sizes, i.e., to intermediate position 604 and contracted position 606.

As per application requirements, if tubing assembly 508 is to be disposed in intermediate position 604 or contracted position 606, protrusion 550 is disengaged from recess 552. Further, first and second adjustment members 538, 540 are pushed towards each other until protrusion 550 engages with recesses 554 or 556 (see FIG. 6B) for disposing tubing assembly 508 in intermediate position 604 or contracted position 606, respectively. It should be noted that when tubing assembly 508 switches to intermediate position 604 from extended position 602, a length of adjustable unit 536 may decrease from maximum length "L4" by a value between 5 mm and 10 mm, or a value between 10 mm and 20 mm. Similarly, when tubing assembly 508 switches to contracted position 606 from extended position 602, the length of adjustable unit 536 may decrease from maximum length "L4" by a value between 10 mm and 20 mm, or a value between 20 mm and 40 mm.

FIG. 7B illustrates tubing assembly 508 in intermediate position 604. For explanatory purposes, only tubular portion 520 and adjustment unit 534 are explained in detail with reference to FIG. 7B. However, it should be noted that details provided herein are equally applicable to tubular portion 522 and adjustment unit 536. Intermediate position 604 may correspond to the intermediate predetermined length, that is smaller than the maximum predetermined length, such that tubing assembly 508 is usable by user 102 (see FIG. 1A) having a "medium" head size. As illustrated in Fig. 7B, in intermediate position 604, adjustable unit 536 has an intermediate length "L5". In intermediate position 604, protrusion 550 (see FIG. 6A) is received within recess 554 (see FIG. 6B).

In one exemplary embodiment, tubing assembly 508 can elongate by one size, or tubing assembly 508 can contract by one size from intermediate position 604. As per application requirements, if tubing assembly 508 is to be disposed in extended position 602 or contracted position 606, protrusion 550 is disengaged from recess 554. Further, for disposing tubing assembly 508 in extended position 602, first and second adjustment members 538, 540 are pulled away from each other until protrusion 550 engages with recess 552 (see FIG. 6B) for disposing tubing assembly 508 in extended position 602. For disposing tubing assembly 508 in contracted position 606, first and second adjustment members 538, 540 are pushed towards each other until protrusion 550 engages with recess 556 (see FIG. 6B) for disposing tubing assembly 508 in contracted position 606. It should be noted that when tubing assembly 508 switches to extended position 602 from intermediate position 604, the length of adjustable unit 536 may increase from intermediate length "L5" by a value between 5 mm and 10 mm, or a value between 10 mm and 20 mm. Similarly, when tubing assembly 508 switches to contracted position 606 from intermediate position 604 , the length of adjustable unit 536 may decrease from intermediate length "L5" by a value between 5 mm and 10 mm, or a value between 10 mm and 20 mm.

FIG. 7C illustrates tubing assembly 508 in contracted position 606. For explanatory purposes, only tubular portion 520 and adjustment unit 534 are explained in detail with reference to FIG. 7C. However, it should be noted that details provided herein are equally applicable to tubular portion 522 and adjustment unit 536. Contracted position 606 may correspond to the minimum predetermined length, that is smaller than the intermediate predetermined length, such that tubing assembly 508 is usable by user 102 (see FIG. 1A) having a "small" head size. As illustrated in Fig. 7C, in contracted position 606, adjustable unit 536 has a minimum length "L6". In contracted position 606, protrusion 550 (see FIG. 6A) is received within recess 556 (see FIG. 6B). In one exemplary embodiment, tubing assembly 508 can elongate up to two sizes, i.e., to intermediate position 604 and extended position 602. As per application requirements, if tubing assembly 508 is to be disposed in intermediate position 604 or extended position 602, protrusion 550 is disengaged from recess 556. Further, first and second adjustment members 538, 540 are pulled away from each other until protrusion 550 engages with recesses 552 or 554 (see FIG. 6B) for disposing tubing assembly 508 in extended position 602 or intermediate position 604, respectively. It should be noted that when tubing assembly 508 switches to intermediate position 604 from contracted position 606, the length of adjustable unit 536 may increase from minimum length "L6" by a value between 5 mm and 10 mm, or a value between 10 mm and 20 mm. Similarly, when tubing assembly 508 switches to extended position 602 from contracted position 606, the length of adjustable unit 536 may increase from minimum length "L6" by a value between 10 mm and 20 mm, or a value between 20 mm and 40 mm.

Referring to FIGS. 7A to 7C, in an example, a difference between maximum length "L4" and intermediate length "L5" is between 5 mm and 10 mm. In another example, the difference between maximum length "L4" and intermediate length "L5" is between 10 mm and 20 mm. In an example, a difference between intermediate length "L5" and minimum length "L6" is between 5 mm and 10 mm. In another example, the difference between intermediate length "L5" and minimum length "L6" is between 10 mm and 20 mm. In an example, a difference between maximum length "L4" and minimum length "L6" is between 10 mm and 20 mm. In an example, the difference between maximum length "L4" and minimum length "L6" is between 20 mm and 40 mm. Since each tubular portion 520, 522 includes respective adjustable unit 534, 536, an overall length of tubing assembly 508 can be adjusted by a total adjustment length that is about twice a difference between maximum length "L4" and minimum length "L6". In an example, the total adjustment length of tubing assembly 508 is between 20 mm and 40 mm. In another example, the total adjustment length of tubing assembly 508 is between 40 mm and 80 mm. In some examples, the total adjustment length of tubing assembly 508 is about 30 mm or about 75 mm.

In various exemplary embodiments, one or more components of tubing assembly 508 may be manufactured using an injection molding process. Further, tubing assembly 508 may be formed from a number of separately formed components that are coupled together via a suitable process.

Referring to FIGS. 1 to 7C, tubing assemblies 108, 508 described herein may comprise fewer components and may be lightweight. Tubing assemblies 108, 508 may not be bulky and may be easy to handle. Further, tubing assemblies 108, 508 may be easy to use as users (e.g., user 102) can easily switch between different discrete positions. Moreover, tubing assemblies 108, 508 may allow the users to select and adjust tubing assemblies 108, 508 to appropriate sizes thereby ensuring an improved fit and comfort. The users may also not be required to determine or calculate any adjustment positions as the discrete positions are predetermined by a manufacturer based on different head sizes. Additionally, the users may not have to assemble or disassemble any component of tubing assemblies 108, 508 for switching between different discrete positions. Further, since first adjustment members 138, 538 are integral with or fixedly connected to corresponding tubular portions 120, 122, 520, 522 and second adjustment members 140, 540 are integral with or fixedly connected to corresponding tubular portions 120, 122, 520, 522, first adjustment members 138, 538 and second adjustment members 140, 540 may not get misplaced and cause an inconvenience to the users.

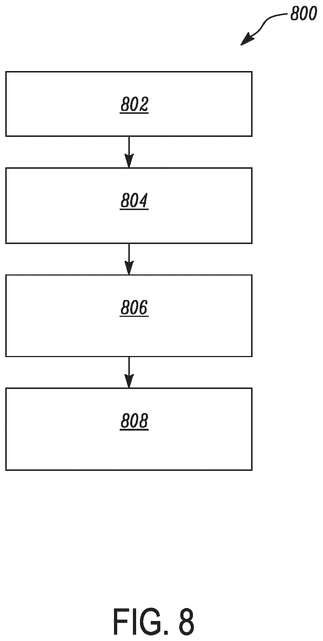

FIG. 8 illustrates a flowchart for a method 800 for delivering the flow of breathing gas to the airway of user 102 (shown in FIG. 1). Method 800 will be described with reference to tubing assembly 108. However, in other exemplary embodiments, method 800 may be applicable to tubing assembly 508.

At step 802, method 800 comprises receiving, via manifold portion 116, the flow of breathing gas from conduit 106. At step 804, method 800 comprises communicating, via tubular portion 120, 122, the flow of breathing gas from manifold portion 116 to patient interface device 110 that is sealingly engaged with airway of user 102. At step 806, method 800 comprises adjusting, via at least one adjustment unit 134, 136, the length of tubular portion 120, 122. At step 808, method 800 comprises selectively and releasably locking at least one adjustment unit 134, 136 at a selected discrete position from the plurality of discrete positions. The plurality of discrete positions corresponds to the plurality of predetermined lengths of tubular portion 120, 122.

There is thus provided a tubing assembly for use with a patient interface device and a method for delivering a flow of breathing gas to an airway of a user, which overcomes the existing problems.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

In the present invention, the expression "at least one of A, B and C" means "A, B, and/or C", and that it suffices if, for example, only B is present. A single element or other unit may fulfill the functions of several items recited in the claims. The mere fact that certain measures are recited in mutually different dependent claims does not indicate that a combination of these measures cannot be used to advantage.

Any reference signs placed between parentheses in the claims should not be construed as limiting the scope of the appended claims.

## Claims

1. A tubing assembly (108) for use with a patient interface device (110) in delivering a flow of breathing gas to an airway of a user, the tubing assembly (108) comprising:
a manifold portion (116) structured to be disposed generally atop the user's head (112) and adapted to be coupled to a conduit carrying the flow of breathing gas;
a plurality of tubular portions (120, 122) made from plastic and/or silicone, each tubular portion (120, 122) extending from the manifold portion (116) to a distal end (124) which is structured to be coupled to the patient interface device (110), each tubular portion (120, 122) being structured to communicate the flow of breathing gas from the manifold portion (116) to the patient interface device (110); and,
at least one adjustment unit (134, 136) configured to adjust a length of a corresponding tubular portion (120, 122) from the plurality of tubular portions (120, 122), wherein the at least one adjustment unit (134, 136) comprises a first adjustment member (138) integral with or fixedly connected to the corresponding tubular portion (122) and a second adjustment member (140) integral with or fixedly connected to the corresponding tubular portion (120, 122), wherein the first adjustment member (138) and the second adjustment member (140) are movable relative to each other, and wherein the first adjustment member (138) and the second adjustment member (140) are selectively and releasably locked to each other at a selected discrete position from a plurality of discrete positions, the plurality of discrete positions corresponding to a plurality of predetermined lengths of the corresponding tubular portion (120, 122),
**characterized in that**
the at least one adjustment unit (134, 136) further comprises an adjustable section (164) integral with the corresponding tubular portion (120, 122), wherein a relative movement between the first adjustment member (138) and the second adjustment member (140) causes the adjustable section (164) to elongate or contract, and wherein the adjustable section (164) is made from plastic and/or silicone.

2. The tubing assembly (108) of claim 1, wherein:
each of the first adjustment member (138) and the second adjustment member (140) extends at least partially along a length of the adjustable section (164);
the first adjustment member (138) is fixedly connected to the corresponding tubular portion (120, 122) proximal to a first end of the adjustable section (164);
the second adjustment member (140) is fixedly connected to the corresponding tubular portion (120, 122) proximal to a second end of the adjustable section (164) opposite to the first end; and
the second adjustment member (140) is spaced apart from the adjustable section (164) and at least partially and slidably received within the first adjustment member (138).

3. The tubing assembly (108) of claim 1, wherein the adjustable section (164) is a corrugated section (172) integrally molded with the corresponding tubular portion (120, 122) and configured to elongate or contract from a normal molded state of the corrugated section (172).

4. The tubing assembly (108) of claim 3, wherein:
the plurality of discrete positions comprises an extended state, a contracted state, and one or more intermediate states between the extended state and the contracted state;
the normal molded state of the corrugated section (172) corresponds to one of the extended state, the contracted state, and the one or more intermediate states; and
the first adjustment member (138) and the second adjustment member (140) are configured to selectively adjust the corrugated section (172) to the other two of the extended state, the contracted state, and the one or more intermediate states.

5. The tubing assembly (108) of claim 1, wherein the first adjustment member (138) comprises a plurality of through apertures (152, 154, 156) corresponding to the plurality of discrete positions, and wherein the second adjustment member (140) comprises a pin (150) selectively, removably, and at least partially receivable within a selected through aperture from the plurality of through apertures (152, 154, 156) in order to releasably lock the second adjustment member (140) to the first adjustment member (138).

6. The tubing assembly (108) of claim 5, wherein the second adjustment member (140) comprises a biasing portion (160) attached to the pin (150) and configured to resiliently bias the pin (150) towards the plurality of through apertures (152, 154, 156).

7. The tubing assembly (108) of claim 1, wherein the corresponding tubular portion (120, 122) and the adjustable section (164) comprise a similar material.

8. The tubing assembly (108) of claim 1, wherein one of the first adjustment member (138) and the second adjustment member (140) is disposed proximal to the manifold portion (116), and the other one of the first adjustment member (138) and the second adjustment member (140) is disposed distal to the manifold portion (116).

9. The tubing assembly (108) of claim 1, wherein the at least one adjustment unit (134, 136) comprises a pair of adjustment units (134, 136) disposed on opposing sides of the manifold portion (116).

10. A respiratory interface system (100) for use in delivering a flow of breathing gas to an airway of a user, the respiratory interface system (100) comprising: a patient interface device (110) structured to sealingly engage the airway of the user; and the tubing assembly (108) of any of claims 1 to 9, wherein the distal end (124) of each tubular portion (120, 122) is coupled to the patient interface device (110).

## Patentansprüche

1. Schlauchanordnung (108) zur Verwendung mit einer Patientenschnittstellenvorrichtung (110) beim Zuführen eines Atemgasstroms zu den Atemwegen eines Benutzers, wobei die Schlauchanordnung (108) Folgendes umfasst:
einen Verteilerabschnitt (116), der strukturiert ist, um im Allgemeinen oben auf dem Kopf (112) des Benutzers angeordnet zu sein und angepasst ist, um an eine Leitung gekoppelt zu werden, die den Atemgasstrom führt;
eine Vielzahl rohrförmiger Abschnitte (120, 122), die aus Kunststoff und/oder Silikon hergestellt sind, wobei sich jeder röhrenförmige Abschnitt (120, 122) von dem Verteilerabschnitt (116) zu einem distalen Ende (124) erstreckt, das strukturiert ist, um mit der Patientenschnittstellenvorrichtung (110) gekoppelt zu sein, wobei jeder röhrenförmige Abschnitt (120, 122) strukturiert ist, um den Atemgasstrom von dem Verteilerabschnitt (116) zu der Patientenschnittstellenvorrichtung (110) zu übertragen; und
mindestens eine Einstelleinheit (134, 136), die konfiguriert ist, um eine Länge eines entsprechenden rohrförmigen Abschnitts (120, 122) aus der Vielzahl rohrförmiger Abschnitte (120, 122) einzustellen, wobei die mindestens eine Einstelleinheit (134, 136) ein erstes Einstellelement (138), das in einem Stück mit dem entsprechenden rohrförmigen Abschnitt (122) oder fest mit diesem verbunden ist, und ein zweites Einstellelement (140) umfasst, das in einem Stück mit dem entsprechenden rohrförmigen Abschnitt (120, 122) oder fest mit diesem verbunden ist, wobei das erste Einstellelement (138) und das zweite Einstellelement (140) in Bezug zueinander bewegbar sind, und wobei das erste Einstellelement (138) und das zweite Einstellelement (140) an einer ausgewählten diskreten Position aus einer Vielzahl diskreter Positionen selektiv und freigebbar miteinander verriegelt sind, wobei die Vielzahl diskreter Positionen einer Vielzahl vorbestimmter Längen des entsprechenden rohrförmigen Abschnitts (120, 122) entspricht,
**dadurch gekennzeichnet, dass**
die mindestens eine Einstelleinheit (134, 136) weiter einen einstellbaren Abschnitt (164) umfasst, der in einem Stück mit dem entsprechenden röhrenförmigen Abschnitt (120, 122) ist, wobei eine relative Bewegung zwischen dem ersten Einstellelement (138) und dem zweiten Einstellelement (140) den einstellbaren Abschnitt (164) veranlasst, sich zu verlängern oder zusammenzuziehen, und wobei der einstellbare Abschnitt (164) aus Kunststoff und/oder Silikon hergestellt ist.

2. Schlauchanordnung (108) nach Anspruch 1, wobei:
sich jedes des ersten Einstellelements (138) und des zweiten Einstellelements (140) mindestens teilweise entlang einer Länge des einstellbaren Abschnitts (164) erstreckt;
das erste Einstellelement (138) fest mit dem entsprechenden rohrförmigen Abschnitt (120, 122) proximal zu einem ersten Ende des einstellbaren Abschnitts (164) verbunden ist;
das zweite Einstellelement (140) fest mit dem entsprechenden rohrförmigen Abschnitt (120, 122) proximal zu einem zweiten Ende des einstellbaren Abschnitts (164), dem ersten Ende entgegengesetzt liegend, verbunden ist; und
das zweite Einstellelement (140) von dem einstellbaren Abschnitt (164) beabstandet ist und mindestens teilweise und verschiebbar innerhalb des ersten Einstellelements (138) aufgenommen ist.

3. Schlauchanordnung (108) nach Anspruch 1, wobei der einstellbare Abschnitt (164) ein gewellter Abschnitt (172) ist, der in einem Stück mit dem entsprechenden rohrförmigen Abschnitt (120, 122) geformt ist und konfiguriert ist, um sich aus einem normalen geformten Zustand des gewellten Abschnitts (172) zu verlängern oder zusammenzuziehen.

4. Schlauchanordnung (108) nach Anspruch 3, wobei:
die Vielzahl diskreter Positionen einen ausgedehnten Zustand, einen zusammengezogenen Zustand und einen oder mehrere Zwischenzustände zwischen dem ausgedehnten Zustand und dem zusammengezogenen Zustand umfasst;
der normale geformte Zustand des gewellten Abschnitts (172) einem des ausgedehnten Zustands, des zusammengezogenen Zustands und einem oder mehreren Zwischenzuständen entspricht; und
das erste Einstellelement (138) und das zweite Einstellelement (140) konfiguriert sind, um den gewellten Abschnitt (172) selektiv in die beiden anderen Zustände, den ausgedehnten Zustand, den zusammengezogenen Zustand und einen oder mehrere Zwischenzustände einzustellen.

5. Schlauchanordnung (108) nach Anspruch 1, wobei das erste Einstellelement (138) eine Vielzahl von Durchgangsöffnungen (152, 154, 156) umfasst, die der Vielzahl diskreter Positionen entsprechen, und wobei das zweite Einstellelement (140) einen Stift (150) umfasst, der selektiv, entfernbar und mindestens teilweise in einer ausgewählten Durchgangsöffnung aus der Vielzahl von Durchgangsöffnungen (152, 154, 156) aufnehmbar ist, um das zweite Einstellelement (140) freigebbar an dem ersten Einstellelement (138) zu verriegeln.

6. Schlauchanordnung (108) nach Anspruch 5, wobei das zweite Einstellelement (140) einen Vorspannabschnitt (160) umfasst, der an dem Stift (150) angebracht ist und konfiguriert ist, um den Stift (150) elastisch in Richtung der Vielzahl von Durchgangsöffnungen (152, 154, 156) vorzuspannen.

7. Schlauchanordnung (108) nach Anspruch 1, wobei der entsprechende rohrförmige Abschnitt (120, 122) und der einstellbare Abschnitt (164) ein ähnliches Material umfassen.

8. Schlauchanordnung (108) nach Anspruch 1, wobei eines des ersten Einstellelements (138) und des zweiten Einstellelements (140) proximal zu dem Verteilerabschnitt (116) angeordnet ist, und das andere des ersten Einstellelements (138) und des zweiten Einstellelements (140) distal zu dem Verteilerabschnitt (116) angeordnet ist.

9. Schlauchanordnung (108) nach Anspruch 1, wobei die mindestens eine Einstelleinheit (134, 136) ein Paar Einstelleinheiten (134, 136) umfasst, die auf entgegengesetzt liegenden Seiten des Verteilerabschnitts (116) angeordnet sind.

10. Beatmungsschnittstellensystem (100) zur Verwendung beim Zuführen eines Atemgasstroms zu den Atemwegen eines Benutzers, wobei das Beatmungsschnittstellensystem (100) Folgendes umfasst: eine Patientenschnittstellenvorrichtung (110), die strukturiert ist, um abdichtend mit den Atemwegen des Benutzers einzugreifen; und die Schlauchanordnung (108) nach einem der Ansprüche 1 bis 9, wobei das distale Ende (124) jedes röhrenförmigen Abschnitts (120, 122) mit der Patientenschnittstellenvorrichtung (110) gekoppelt ist.

## Revendications

1. Ensemble tubulure (108) pour utilisation avec un dispositif d'interface patient (110) pour distribuer un flux de gaz respiratoire aux voies respiratoires d'un utilisateur, l'ensemble tubulure (108) comprenant :
une partie collecteur (116) structurée pour être disposée généralement au-dessus de la tête (112) de l'utilisateur et conçue pour être couplée à un conduit transportant le flux de gaz respiratoire ;
une pluralité de parties tubulaires (120, 122) en plastique et/ou silicone, chaque partie tubulaire (120, 122) s'étendant de la partie collecteur (116) à une extrémité distale (124) qui est structurée pour être couplée au dispositif d'interface patient (110), chaque partie tubulaire (120, 122) étant structurée pour communiquer le flux de gaz respiratoire de la partie collecteur (116) au dispositif d'interface patient (110) ; et,
au moins une unité d'ajustement (134, 136) configurée pour ajuster une longueur d'une partie tubulaire correspondante (120, 122) parmi la pluralité de parties tubulaires (120, 122), dans lequel la au moins une unité d'ajustement (134, 136) comprend un premier élément d'ajustement (138) solidaire de ou relié à demeure à la partie tubulaire correspondante (122) et un second élément d'ajustement (140) solidaire de ou relié à demeure à la partie tubulaire correspondante (120, 122), dans lequel le premier élément d'ajustement (138) et le second élément d'ajustement (140) sont mobiles l'un par rapport à l'autre, et dans lequel le premier élément d'ajustement (138) et le second élément d'ajustement (140) sont verrouillés l'un à l'autre de manière sélective et libérable dans une position distincte sélectionnée parmi une pluralité de positions distinctes, la pluralité de positions distinctes correspondant à une pluralité de longueurs prédéterminées de la partie tubulaire correspondante (120, 122),
**caractérisé en ce que**
la au moins une unité d'ajustement (134, 136) comprend en outre une section ajustable (164) solidaire de la partie tubulaire correspondante (120, 122), dans lequel un mouvement relatif entre le premier élément d'ajustement (138) et le second élément d'ajustement (140) provoque l'allongement ou la contraction de la section ajustable (164), et dans lequel la section ajustable (164) est en plastique et/ou silicone.

2. Ensemble tubulure (108) selon la revendication 1, dans lequel :
chacun du premier élément d'ajustement (138) et du second élément d'ajustement (140) s'étend au moins partiellement le long d'une longueur de la section ajustable (164) ;
le premier élément d'ajustement (138) est relié à demeure à la partie tubulaire correspondante (120, 122) à proximité d'une première extrémité de la section ajustable (164) ;
le second élément d'ajustement (140) est relié à demeure à la partie tubulaire correspondante (120, 122) à proximité d'une seconde extrémité de la section ajustable (164) opposée à la première extrémité ; et
le second élément d'ajustement (140) est espacé de la section ajustable (164) et reçu au moins partiellement et de manière coulissante à l'intérieur du premier élément d'ajustement (138).

3. Ensemble tubulure (108) selon la revendication 1, dans lequel la section ajustable (164) est une section ondulée (172) moulée d'un seul tenant avec la partie tubulaire correspondante (120, 122) et configurée pour s'allonger ou se contracter à partir d'un état moulé normal de la section ondulée (172).

4. Ensemble tubulure (108) selon la revendication 3, dans lequel :
la pluralité de positions distinctes comprennent un état étendu, un état contracté et un ou plusieurs états intermédiaires entre l'état étendu et l'état contracté ;
l'état moulé normal de la section ondulée (172) correspond à l'un parmi l'état étendu, l'état contracté et les un ou plusieurs états intermédiaires ; et
le premier élément d'ajustement (138) et le second élément d'ajustement (140) sont configurés pour ajuster sélectivement la section ondulée (172) aux deux autres parmi l'état étendu, l'état contracté et les un ou plusieurs états intermédiaires.

5. Ensemble tubulure (108) selon la revendication 1, dans lequel le premier élément d'ajustement (138) comprend une pluralité d'ouvertures traversantes (152, 154, 156) correspondant à la pluralité de positions distinctes, et dans lequel le second élément d'ajustement (140) comprend un ergot (150) pouvant être reçu de manière sélective, amovible et au moins partiellement dans une ouverture traversante sélectionnée parmi la pluralité d'ouvertures traversantes (152, 154, 156) afin de verrouiller de manière libérable le second élément d'ajustement (140) sur le premier élément d'ajustement (138).

6. Ensemble tubulure (108) selon la revendication 5, dans lequel le second élément d'ajustement (140) comprend une partie de sollicitation (160) fixée à l'ergot (150) et configurée pour solliciter de manière élastique l'ergot (150) vers la pluralité d'ouvertures traversantes (152, 154, 156).

7. Ensemble tubulure (108) selon la revendication 1, dans lequel la partie tubulaire correspondante (120, 122) et la section ajustable (164) comprennent un matériau similaire.

8. Ensemble tubulure (108) selon la revendication 1, dans lequel l'un du premier élément d'ajustement (138) et du second élément d'ajustement (140) est disposé à proximité de la partie collecteur (116), et l'autre du premier élément d'ajustement (138) et du second élément d'ajustement (140) est disposé de manière distale par rapport à la partie collecteur (116).

9. Ensemble tubulure (108) selon la revendication 1, dans lequel la au moins une unité d'ajustement (134, 136) comprend une paire d'unités d'ajustement (134, 136) disposées sur des côtés opposés de la partie collecteur (116).

10. Système d'interface respiratoire (100) pour utilisation dans le distribution d'un flux de gaz respiratoire aux voies respiratoires d'un utilisateur, le système d'interface respiratoire (100) comprenant : un dispositif d'interface patient (110) structuré pour venir en prise de manière étanche avec les voies respiratoires de l'utilisateur ; et l'ensemble tubulure (108) selon l'une quelconque des revendications 1 à 9, dans lequel l'extrémité distale (124) de chaque partie tubulaire (120, 122) est couplée au dispositif d'interface patient (110).
